(19) Europäisches Patentamt

European Patent Office

Office européen des brevets

(11) **EP 1 179 061 B1**

(12) **EUROPEAN PATENT SPECIFICATION**

(45) Date of publication and mention
of the grant of the patent:
**20.09.2006 Bulletin 2006/38**

(51) Int Cl.:
*C12N 15/12* (2006.01)    *C12N 15/62* (2006.01)
*C07K 14/78* (2006.01)    *C07K 19/00* (2006.01)
*C12N 15/861* (2006.01)    *C12N 15/867* (2006.01)
*A61K 48/00* (2006.01)    *G01N 33/68* (2006.01)

(21) Application number: **00930428.8**

(22) Date of filing: **05.05.2000**

(86) International application number:
**PCT/US2000/012392**

(87) International publication number:
**WO 2000/068379 (16.11.2000 Gazette 2000/46)**

(54) **METHODS FOR TREATING TUMORS USING ANTIANGIOGENIC COMPOUNDS**

VERFAHREN ZUR BEHANDLUNG VON TUMOREN MITTELS ANTIANGIOGENER SUBSTANZEN

PROCEDE DE TRAITEMENT DE TUMEURS A L'AIDE DE COMPOSES ANTIANGIOGENIQUES

(84) Designated Contracting States:
**AT BE CH CY DE DK ES FI FR GB GR IE IT LI LU MC NL PT SE**

(30) Priority: **07.05.1999 US 133243 P**

(43) Date of publication of application:
**13.02.2002 Bulletin 2002/07**

(73) Proprietor: **THE GOVERNMENT OF THE UNITED STATES OF AMERICA, as represented by THE SECRETARY, DEPARTMENT OF HEALTH AND HUMAN SERVICES Rockville, MD 20852 (US)**

(72) Inventors:
• **LIBUTTI, Steven, K.**
**North Potomac, MD 20878 (US)**
• **FELDMAN, Andrew**
**Greenbelt, MD 20770 (US)**

(74) Representative: **Leissler-Gerstl, Gabriele Eisenführ, Speiser & Partner, Patentanwälte, Arnulfstrasse 25 80335 München (DE)**

(56) References cited:
**WO-A1-97/16169        WO-A1-98/48834**
**WO-A2-97/34586        WO-A2-98/49321**

• **TANAKA T. ET AL.: "VIRAL VECTOR-TARGETED ANTIANGIOGENIC GENE THERAPY UTILIZING AN ANGIOSTATIN COMPLEMENTARY DNA" CANCER RESEARCH, vol. 58, no. 15, 1 August 1998 (1998-08-01), pages 3362-3369, XP000857409 ISSN: 0008-5472**
• **BRAMSON J. L. ET AL.: "Direct intratumoral injection of an adenovirus expressing Interleukin-12 induces regression and long-lasting immunity that is associated with highly localized expression of Interleukin-12" HUMAN GENE THERAPY, vol. 7, 20 October 1996 (1996-10-20), pages 1995-2002, XP002093895 ISSN: 1043-0342**
• **CRYSTAL R. G. : "The body as manufacturer of endostatin." NATURE BIOTECHNOLOGY, vol. 17, April 1999 (1999-04), pages 336-337, XP002145429 cited in the application**
• **TANAKA T. ET AL.: "Viral vector-mediated transduction of a modified platelet factor 4 cDNA inhibits angiogenesis and tumor growth." NATURE MEDICINE, vol. 3, no. 4, 1997, pages 437-442, XP002145430 ISSN: 1078-8956**
• **BLEZINGER P. ET AL.: "SYSTEMIC INHIBITION OF TUMOR GROWTH AND TUMOR METASTASES BY INTRAMUSCULAR ADMINISTRATION OF THE ENDOSTATIN GENE" NATURE BIOTECHNOLOGY, vol. 17, no. 4, April 1999 (1999-04), pages 343-348, XP000857410 ISSN: 1087-0156 cited in the application**

- SCHWARZ M. ET AL.: "EMAP II: A modulator of neovascularization in the developing lung." AMERICAN JOURNAL OF PHYSIOLOGY, vol. 20, no. 2, February 1999 (1999-02), pages L365-L375, XP002145431 ISSN: 1081-5589
- PIKE S. E. ET AL.: "Vasostatin, a calreticulin fragment, inhibits angiogenesis and suppresses tumor growth." JOURNAL OF EXPERIMENTAL MEDICINE, vol. 188, no. 12, 21 December 1998 (1998-12-21), pages 2349-2356, XP002145432 ISSN: 0022-1007
- SHUTTLEWORTH C. A.: "Type VIII collagen." INTERNATIONAL JOURNAL OF BIOCHEMISTRY & CELL BIOLOGY, vol. 29, no. 10, October 1997 (1997-10), pages 1145-1148, XP000938722 ISSN: 1357-2725
- RAMCHANDRAN R. ET AL.: "Antiangiogenic activity of restin, NC10 domain of human collagen XV: Comparison to endostatin." BIOCHEMICAL AND BIOPHYSICAL RESEARCH COMMUNICATIONS, vol. 255, no. 3, 24 February 1999 (1999-02-24), pages 735-739, XP002145434 ISSN: 0006-291X
- RAMCHANDRAN R. ET AL.: "Cloning, expression of a novel anti-angiogenic protein: Restatin." PROCEEDINGS OF THE AMERICAN ASSOCIATION FOR CANCER RESEARCH, vol. 40, March 1999 (1999-03), page 620 XP000938553 ISSN: 0197-016X
- SAUTER B. V. ET AL.: "Adenovirus-mediated gene transfer of endostatin in vivo results in high level of transgene expression and inhibition of tumor growth and metastases." PROCEEDINGS OF THE NATIONAL ACADEMY OF SCIENCES OF THE UNITED STATES, vol. 97, no. 9, 25 April 2000 (2000-04-25), pages 4802-4807, XP002145436 April 25, 2000 ISSN: 0027-8424

SEQUENCE LISTING

<110> Libutti, Steven K. Feldman, Andrew

<120> METHODS FOR TREATING TUMORS USING ANTIANGIOGENIC COMPOUNDS

<130> 14014.0322

<160> 7

<170> FastSEQ for Windows Version 3.0

<210> 1
<211> 39
<212> DNA
<213> Artificial Sequence

<220>
<223> Description of Artificial Sequence:/Note = synthetic construct; forward primer

<400> 1
gatctctaga ccaccatgca tactcatcag gactttcag          39

<210> 2
<211> 30
<212> DNA
<213> Artificial Sequence

<220>
<223> Description of Artificial Sequence:/Note = synthetic construct; reverse primer

<400> 2
gatcatcgat ctatttggag aaagaggtca          30

<210> 3
<211> 40
<212> DNA
<213> Artificial Sequence

<220>
<223> Description of Artificial Sequence:/Note = synthetic construct; forward primer

<400> 3
gatctctaga ccaccatggt tgcgctcaac agccccctgt          40

<210> 4
<211> 37
<212> DNA
<213> Artificial Sequence

<220>
<223> Description of Artificial Sequence:/Note = synthetic construct; reverse primer

<400> 4
gatcatcgat ctactacttg gaggcagtca tgaagct          37

<210> 5
<211> 90
<212> DNA
<213> Artificial Sequence

<220>
<223> Description of Artificial Sequence:/Note = synthetic construct

<400> 5

```
gatctctaga ccaccatgag gtacatgatt ttaggcttgc tcgcccttgc ggcagtctgc   60
agcgcggccc atactcatca ggactttcag                                    90
```

<210> 6
<211> 90
<212> DNA
<213> Artificial Sequence

<220>
<223> Description of Artificial Sequence:/Note = synthetic construct

<400> 6

```
gatctctaga ccaccatgag gtacatgatt ttaggcttgc tcgcccttgc ggcagtctcc   60
agcgcggccg ttgcgctcaa cagccccctg                                    90
```

<210> 7
<211> 18
<212> PRT
<213> Artificial Sequence

<220>
<223> Description of Artificial Sequence:/Note = synthetic construct

<400> 7

```
Met Arg Tyr Met Ile Leu Gly Leu Leu Ala Leu Ala Ala Val Cys Ser
 1               5                 10                  15
Ala Ala
```

Description

BACKGROUND OF THE INVENTION

FIELD OF THE INVENTION

This invention relates generally to antiangiogenic compounds and compositions and methods utilizing antiangiogenic compounds and compositions. Specifically, this invention relates to methods of producing antiangiogenic compounds, methods of delivering antiangiogenic compounds, methods of treatment using antiangiogenic compounds, and methods of screening antiangiogenic compounds for bioactivity using a virus expression system.

BACKGROUND ART

Angiogenesis is now recognized as a critical process in tumor progression and is required for expansive growth of solid tumors. The transition from a non-angiogenic to an angiogenic phenotype and subsequent tumor vascularization likely involves positive and negative regulators of angiogenesis (*Gibaldi M.* 1998. " Regulating angiogenesis: a new therapeutic strategy" *J. Clinic. Pharmacol.* 38:898-903.) The switch to angiogenesis is usually accomplished by a subset of tumor cells within the *in situ* lesion. The new microvessels that are recruited provide a neovascular meshwork which supports the growth, and facilitates invasion and metastasis of the rapidly expanding tumor mass (*O'Reilly et al.* 1994. "Angiostatin: A Circulating Endothelial Cell Inhibitor That Suppresses Angiogenesis and Tumor Growth." Cold Spring Harbor Symposium on Quantitative Biology, LIX:471-482). Regulation of vascular growth also involves complex interactions of extracellular matrix molecules, proteolytic enzymes and cell adhesion molecules. Each step in the angiogenic process represents a potential target for therapeutic anticancer strategies.

Endostatin is a protein derived from the cleavage of the precursor collagen XVIII. It is an endogenous inhibitor of angiogenesis and tumor growth that can completely or nearly completely inhibit angiogenesis and can induce dormancy or regression of large tumors in mice (*Wu et al.* 1997. "Suppression of Tumor Growth with Recombinant Murine Angiostatin" Biochem and Biophys. Res. Comm. 236:651-654). Systematic administration of endostatin to tumor bearing animals results in the regression of blood vessels leading to the regression of the tumors. Furthermore, endostatin, does not induce acquired drug resistance, a problem associated with chemotherapy and other cytochemical therapies (*Boehm et al.* 1997. "Antiangiogenic therapy of experimental cancer does not induce acquired drug resistance" Nature 390: 404-407). However, difficulties in producing sufficient recombinant endostatin for widespread clinical use has presented a significant obstacle in developing an endostatin therapy model.

Viral vectors comprising antiangiogenic proteins have been disclosed in the prior art. For example from WO 98/49321 A a replication deficient adenovirus vector was known. Moreover, T. TANAKA et al reported about viral vector targeted antiangiogenic gene therapy utilizing an angiostatin complementary DNA in Cancer Research, Vol. 58, No. 15, pages 3362-3369 (1 August 1998). J. L. BRAM-SON et al published an article entitled "Direct intratumoral injection of an adenovirus expressing Interleukin-12 induces regression and long-lasting immunity that is associated with highly localized expression of Interleukin-12" in HUMAN GENE THERAPY, Vol. 7, pages 1995-2002 (20 October 1996). The inhibition of angiogenesis and tumor growth by a viral vector mediated transduction of a modified platelet vector for cDNA has been reported by T. TANAKA et al in NATURE MEDICINE, Vol. 3, No. 4, pages 437-442 (1997).

Moreover, antiangiogenic vectors have been described in several documents, for example by M. SCHWARZ et al in "EMAP II: A modulator of neovascularization in the developing lung" AMERICAN JOURNAL OF PHYSIOLOGY, Vol. 20, No. 2, pages L365-L375 (February 1999); S. E. PIKE et al "Vasostatin, a calreticulin fragment, inhibits angiogenesis and suppresses tumor growth" JOURNAL OF EXPERIMENTAL MEDICINE, Vol. 188, No. 12, pages 2349-2356 (21 December 1998); WO 97/34586 A; by C.A. SHUTTLEWORTH in "Type VIII collagen" INTERNATIONAL JOURNAL OF BIOCHEMISTRY & CELL BIOLOGY", Vol. 29, No. 10, pages 1145-1148 (October 1997); and in two articles by R. RAMCHANDRAN et al entitled: "Antiangiogenic activity of restin, NC10 domain of human collagen XV: Comparison to endostatin" BIOCHEMICAL AND BIOPHYSICAL RESEARCH COMMUNICATIONS, Vol. 255, No. 3, pages 735-739 (24 February 1999); and "Cloning, expression of a novel anti-angiogenic protein: Restatin" PROCEEDINGS OF THE AMERICAN ASSOCIATION FOR CANCER RESEARCH, Vol. 40, page 620 (March 1999). However, none of these documents disclosed a viral vector that yielded expression of an antiangiogenic protein at circulating levels sufficient to treat a tumor.

The present invention provides an improved method for delivering endostatin to cells by administering an adenovirus vector carrying an endostatin gene. Another unique feature of this adenoviral construct is an 18 amino acid E3/19K adenoviral signal sequence that allows secretion of endostatin from cells infected with the adenoviral vector. The methods described herein disclose a system that provides virus vectors comprising nucleic acids encoding endostatin to provide an efficient means of gene transfer into a variety of cell types. Additionally, virus-mediated gene transfer results in high level protein expression. This combination of facile gene transfer and high specific activity provides a rapid means of producing significant quantities of recombinant molecules, either for subsequent administration to a host or for production of endostatin within a host. The ability to produce secretory endostatin allows production of soluble endostatin at the site of adenovirus infection which can circulate throughout the body, allowing for more efficient treatment strategies. Thus, the present methods also provide a much-needed, improved therapeutic method for use in treating tumors that avoids the adverse side effects of previous treatments.

## SUMMARY OF THE INVENTION

In accordance with the purpose(s) of this invention, as embodied and broadly described herein, this invention, in one aspect, provides a compound comprising a recombinant nucleic acid encoding an antiangiogenic protein operatively linked to an adenovirus E3/K19 signal sequence inserted within a viral nucleic acid, wherein the recombinant nucleic acid can be packaged in a virus particle and wherein expression of the recombinant nucleic acid encoding the antiangiogenic protein results in production of the antiangiogenic protein.

The present invention also discloses a method of delivering an antiangiogenic protein to a cell comprising administering to the cell a virus comprising a recombinant nucleic acid encoding an antiangiogenic protein inserted within the viral nucleic acid, wherein the recombinant nucleic acid can be packaged in a virus particle, and wherein expression of the recombinant nucleic acid encoding the antiangiogenic protein results in production of the antiangiogenic protein, thereby delivering the antiangiogenic protein to the cell.

The invention also discloses a method of delivering an antiangiogenic protein to a subject comprising administering to the subject a virus comprising a recombinant nucleic acid encoding an antiangiogenic protein inserted within the viral nucleic acid, wherein the recombinant nucleic acid can be packaged in a virus particle, and whereby a cell of the subject expresses the recombinant nucleic acid encoding the antiangiogenic protein, thereby delivering the antiangiogenic protein to the subject.

The present invention also discloses a method of treating a tumor in a subject comprising administering to the subject a virus comprising a recombinant nucleic acid encoding an antiangiogenic protein inserted within the viral nucleic acid, wherein the recombinant nucleic acid can be packaged in a virus particle, and whereby a cell in the subject expresses the recombinant nucleic acid encoding the antiangiogenic protein and produces the antiangiogenic, thereby treating the tumor.

Also provided is a method of producing an antiangiogenic protein comprising administering to a cell a virus comprising a recombinant nucleic acid encoding an antiangiogenic protein inserted within the viral nucleic acid, wherein the recombinant nucleic acid can be packaged in a virus particle, and whereby expression of the recombinant nucleic acid produces the antiangiogenic protein.

Also provided is a method of screening an antiangiogenic protein for bioactivity, comprising administering to a first cell a virus containing a recombinant nucleic acid encoding the antiangiogenic protein, wherein the first cell expresses the recombinant nucleic acid encoding the antiangiogenic protein and thereby produces the antiangiogenic protein, contacting a second cell with the antiangiogenic protein, and monitoring the second cell for a biological response to the antiangiogenic protein, thereby screening the antiangiogenic protein for bioactivity.

## BRIEF DESCRIPTION OF THE DRAWINGS

Figure 1 shows the SKL-35 plasmid constructed from pSSmendo-6 (murine endostatin with Kozack and E19 signal sequence) and pAdCMV.

Figure 2 shows the SKL-22 plasmid constructed from pkmendo-2 (murine endostatin with Kozack) and pAdCMV.

Figure 3 shows the anti-tumor effect of Ad-ss-mEndo in vivo.

Figure 4 shows the supernatant endostatin levels after adenoviral infection of 293 cells.

Figure 5 shows the supernatant endostatin levels after adenoviral infection of 293 cells.

Figure 6 shows the inhibition of lung microvascular endothelial cells following Ad-ss-mEndo vs. Ad-Luc viral supernatant incubation.

Figure 7 shows the plasma endostatin levels in nude mice 5 days after $10^9$ pfu adenoviral intraperitoneal injection.

Figure 8a is a schematic of an adenoviral construct containing a murine endostatin gene.

Figure 8b shows 293 cells infected with Ad-ss-mEndo or control adenovirus (Ad-luc) at varying multiplicities of infection, yielding dose-dependent supernatant endostatin concentrations up to 920± 33 ng/mL after Ad-ss-Endo infection.

Figure 8c shows a western blot of supernatant from cells where cells infected with Ad-ss-mEndo (lane 7) but not Ad-luc (lane 6) demonstrated an appropriate 20kD band. Lanes 1-5, two-fold dilutions of recombinant murine endostatin (1000 ng/mL to 62.5 ng/mL).

Figure 9a shows that human melanoma cells infected with Ad-ss-Endo generated dose-dependent endostatin concentrations in supernatant.

Figure 9b shows that five-fold dilutions of supernatants inhibited proliferation of bovine endothelial cells up to 61 ± 4% compared to supernatant from uninfected cells.

Figure 10 illustrates plasma endostatin levels 2 to 13 days after a single intravenous injection of Ad-ss-mEndo. Levels were 1770 ± 292 ng/ml at 4 days, and remained significantly higher than controls at day 13.

Figure 11a shows that murine hepatocytes are readily infected by Ad-β-gal.

Figure 11b shows that MC38 tumor cells are relatively resistant to adenoviral infection at the same multiplicity of infection.

Figure 11c shows that by the end of a 2-week treatment experiment, tumors in mice receiving Ad-ss-mEndo were 40% smaller than controls.

Figure 12 shows that IV administration of an adenoviral vector carrying the endostatin gene results in high endostatin levels (>2000 ng/ml).

Figure 13 shows retroviral transduction of neoplastic murine hepatocytes with the endostatin gene leads to secretion of endostatin sufficiently active to inhibit tumor growth.

## DETAILED DESCRIPTION OF THE INVENTION

The present invention may be understood more readily by reference to the following detailed description of the preferred embodiments of the invention and the Example included therein.

It must be noted that, as used in the specification and the appended claims, the singular forms "a," "an," and "the" include plural referents unless the context clearly dictates otherwise. For example, a cell can mean a single cell of a population of cells.

The present invention provides a compound comprising a recombinant nucleic acid encoding an antiangiogenic protein operatively linked to an adenovirus E3/K19 signal sequence inserted within a viral nucleic acid, wherein the recombinant nucleic acid can be packaged in a virus particle and wherein expression of the recombinant nucleic acid encoding the antiangiogenic protein results in production of the antiangiogenic protein.

Therefore, in one of its most general applications, the invention relates to a recombinant virus incorporating a DNA segment having a sequence encoding an antiangiogenic protein. The nucleic acid encoding the antiangiogenic protein is then coupled with a portion of a viral nucleic acid sufficient to allow the nucleic acid to be packaged in a viral particle. The antiangiogenic protein and therefore the nucleic acid encoding the antiangiogenic protein of the invention may be any of a number of such antiangiogenic proteins known in the art. An exemplary list of certain antiangiogenic factors includes, but is not limited to, endostatin, pigment epithelium-derived growth factor (PEDF), endothelial monocyte activating peptide II, canstatin, interferon-inducible protein-10, thrombospondin, EMAP-II, IP-10, angiostatin, vasostatin, vasculostatin, IL-12, platelet factor 4, cleavage products of collagen VIII, cleavage products of collagen XV such as restin (*Ramchandran et al.* 1999 "Antiangiogenic activity of restin, NC10 domain of human collagen XV: comparison to endostatin" Biochem. Biophys. Res. Commun. 24;255(3):735-739), and restatin.

One skilled in the art will appreciate that the viral vector used in the methods claimed herein and as part of the compounds claimed herein can comprise any viral vector amenable to production of the antiangiogenic compound or one that can be used to produce an antiangiogenic compound. For example, the virus can comprise recombinant adenovirus vectors *Mitani et al.* "Transduction of human bone marrow by adenoviral vector." Human Gene Therapy 5:

941-948 (1994)), adenoassociated viral vectors (*Goodman et al.* "Recombinant adenoassociated virus-mediated gene transfer into hematopoietic progenitor cells." Blood 84:1492-1500 (1994)), lentiviral vectors (*Naidini et al.* "In vivo gene delivery and stable transduction of nondividing cells by a lentiviral vector." Science 272:263-267 (1996)), pseudotyped retroviral vectors (*Agrawal et al.* "Cell-cycle kinetics and VSV-G pseudotyped retrovirus mediated gene transfer in blood-derived CD34$^+$ cells." Exp. Hematol. 24:738-747 (1996)), vaccinia vectors, and physical transfection techniques (*Schwarzenberger et al.* "Targeted gene transfer to human hematopoietic progenitor cell lines through the *c-kit* receptor." Blood 87:472-478 (1996)). This invention can be used in conjunction with any of these or other commonly used gene transfer methods. In a preferred embodiment of the present invention, the specific vector for delivering the nucleic acid encoding a antiangiogenic protein comprises an adenovirus vector or a retrovirus vector.

The retrovirus can be one found in the oncovirinae subfamily of retroviruses, such as one from HTLV-I or HTLV-II (human T-cell leukemia virus type I and type II, respectively). Additionally, the retrovirus can be one from the lentivirinae subfamily of retroviruses, such as HIV-1, HIV-II, SIV, FIV, EIAV, and CAEV (human immunodeficiency virus type I, human immunodeficiency virus type II, simian immunodeficiency virus, feline immunodeficiency virus, equine infectious anemia virus, and caprine arthritis-encephalitis virus, respectfully).

The nucleic acid encoding the antiangiogenic protein can be positioned within any location of the genome of the virus wherein the virus genome with this sequence encoding an antiangiogenic protein may still be packaged into a virus particle. For example, *Ghosh-Choudhury et al.* have indicated that the maximum amount of nucleic acid that adenovirus can package into viral capsids is approximately 2000 bases in excess of the wild-type genome. (*Ghosh-Choudhury et al.,* (1987) EMBO J. 6:1733-1739). One can therefore position the antiangiogenic protein-encoding nucleic acid within or in replacement of a region of the E1 region of adenovirus, for example, to disrupt the E1 gene and therefore inactivate the cellular transforming capacity of this adenoviral gene, as well as enable the recombinant virus to express and therefore produce the desired antiangiogenic protein. The minimum amount of adenoviral nucleic acid in these constructs is that amount that will allow the recombinant adenoviral-antiangiogenic nucleic acid to be packaged. Additionally, the site of insertion of the nucleic acid encoding the antiangiogenic protein into the adenoviral genome, or portion thereof, is selected as to allow the final recombinant nucleic acid to be packaged, as is known to one skilled in the art.

The term "adenoviral genome" or "adenovirus genome" is used herein to describe an adenoviral nucleic acid that is capable of being packaged into an adenovirus particle. Therefore this nucleic acid may comprise an entire wild-type adenoviral genome or a mutant thereof, or a construct wherein the only adenoviral sequences present are those which enable the nucleic acid to be packaged into an adenovirus particle, or any variation thereof. Packageable lengths of nucleic acids for specific antiangiogenic protein are known in the art. This adenoviral genome can be coupled with any desired nucleic acid insert, such as an antiangiogenic protein, such that the adenoviral genome, when packaged into an adenovirus particle, also packages the nucleic acid insert. One skilled in the art will appreciate that the nucleic acid insert combined with the adenoviral nucleic acid will be a total nucleic acid length that will allow the total nucleic acid to be packaged into an adenovirus particle.

By "compound comprising a recombinant nucleic acid" is meant that the nucleic can be that commonly referred to as a nucleic acid, but this compound, for example, can also be a derivative of a typical nucleic acid such as nucleic acids which are modified to contain a terminal or internal reporter molecule or those nucleic acids containing non-typical bases or sugars. These reporter molecules include, but are not limited to isotopic and non-isotopic reporter molecules. Examples of non-isotopic reporter molecules include, but are not limited to biotin, LC-biotin, fluorescein, acridine, cholesterol, and dinitrophenyl labels which can be attached to a 2-aminobutyl-1,3-propanediol backbone (Clontech, Palo Alto, CA).

One skilled in the an will appreciate that there are numerous techniques available by which one can obtain a nucleic acid sequence encoding an antiangiogenic protein. One example of a method of obtaining the nucleic acid is by constructing the nucleic acid by synthesizing a recombinant DNA molecule. For example, oligonucleotide synthesis procedures are routine in the art and oligonucleotides coding for a particular protein or regulatory region are readily obtainable through automated DNA synthesis. A nucleic acid for one strand of a double-stranded molecule can be synthesized and hybridized to its complementary strand. One can design these oligonucleotides such that the resulting double-stranded molecule has either internal restriction sites or appropriate 5' or 3' overhangs at the termini for cloning into an appropriate vector. Double-stranded molecules coding for relatively large proteins or regulatory regions can be synthesized by first constructing several different double-stranded molecules that code for particular regions of the protein or regulatory region, followed by ligating these DNA molecules together. For example, *Cunningham, et al.,* "Receptor and Antibody Epitopes in Human Growth Hormone Identified by Homolog-Scanning Mutagenesis" *Science,* Vol. 243, pp. 1330-1336 (1989), have constructed a synthetic gene encoding the human growth hormone gene by first constructing overlapping and complementary synthetic oligonucleotides and ligating these fragments together. See also, *Ferretti, et al.,* Proc. Nat. Acad. Sci. 82:599-603 (1986), wherein synthesis of a 1057 base pair synthetic bovine rhodopsin gene from synthetic oligonucleotides is disclosed. Once the appropriate DNA molecule is synthesized, this DNA can be cloned downstream of an appropriate promoter. Techniques such as this are routine in the art and are well documented.

Another example of a method of obtaining a sequence encoding an antiangiogenic protein is to utilize traditional recombinant techniques to generate the sequence from the native sources of the individual components. One can isolate the corresponding wild-type nucleic acid for part or all of the antiangiogenic protein from the organism in which it is found and clone it in an appropriate vector. For example, a DNA or cDNA library can be constructed and screened for the presence of the nucleic acid of interest. Methods of constructing and screening such libraries are well known in the art and kits for performing the construction and screening steps are commercially available (for example, Stratagene Cloning Systems, La Jolla, CA). Once isolated, the nucleic acid encoding the antiangiogenic protein can be subsequently cloned into an appropriate vector, or if necessary, be modified to facilitate the subsequent cloning steps. Such modification steps are routine, an example of which is the addition of oligonucleotide linkers which contain restriction sites to the termini of the nucleic acid. Such modification steps may be utilized to facilitate insertion of the sequence encoding an antiangiogenic protein into a viral genome, such as an adenoviral genome or a retroviral genome. General methods for these and other cloning procedures are set forth in *Sambrook et al.,* "Molecular Cloning, a Laboratory Manual" Cold Spring Harbor Laboratory Press (1989). Once isolated, the sequence encoding an antiangiogenic protein can also be modified for other purposes such as increased expression by altering specific codons, for example, or for increased binding to a receptor or a ligand.

Yet another example of a method of obtaining a sequence encoding an antiangiogenic protein is to amplify the corresponding wild-type nucleic acid from the nucleic acids found within a host organism containing the wild-type nucleic acid and clone the amplified nucleic acid in an appropriate vector. One skilled in the art will appreciate that the amplification step may be combined with a mutation step, if desired, using primers not completely homologous to the target nucleic acid for example, to simultaneously amplify the nucleic acid and alter specific positions of the nucleic acid.

The nucleic acid encoding the antiangiogenic protein, however obtained, if not already in the context of a viral genome, can be inserted into the selected viral genome. Once the sequence encoding an antiangiogenic protein is cloned into the viral genome, or part of the viral genome, this nucleic acid will generally be constructed such that the sequence encoding the antiangiogenic protein is positioned adjacent to and under the control of an effective promoter. The promoter can be selected based upon the ultimate cell in which expression is desired. In certain cases, the promotor may comprise a prokaryotic promoter where the sequence encoding the antiangiogenic protein is being adapted for expression in a prokaryotic host as well as in a eukaryotic vector. For example, the sequence encoding the antiangiogenic protein may be expressed in a prokaryotic host under the direction of one promoter, while the same sequence encoding the antiangiogenic protein may be expressed in a eukaryotic host under the direction of a eukaryotic promoter in the same construct. In other cases, the promoter may comprise only a eukaryotic promoter where the vector is being specifically adapted for expression in a eukaryotic host. Promoters of particular utility in the vectors of the invention comprise cytomegalovirus promoters and adenoviral promoters. Furthermore, an inducible promoter, such as a heat shock promoter, a metallothionein promoter, a lac-inducible promoter, a tetracycline-inducible promoter, or a repressible promoter can be used. Regardless of the exact nature of the antiangiogenic protein's promoters, the recombinant virus of the present invention will incorporate a nucleic acid segment encoding an antiangiogenic protein as described herein.

The sequence encoding the antiangiogenic protein inserted within the viral genome can be positioned such that a virus promoter is operatively linked to the antiangiogenic protein insert in which viral promoter can then direct transcription of the sequence encoding the antiangiogenic protein, or the sequence encoding the antiangiogenic protein may contain its own promoter. Similarly, the sequence encoding the antiangiogenic protein may be positioned wherein the nucleic acid encoding the antiangiogenic protein may utilize other viral regulatory regions or sites such as splice junctions and polyadenylation signals and/or sites. Alternatively, the nucleic acid encoding an antiangiogenic protein may contain a different promoter or other regulatory sequences, such as splice sites and polyadenylation sequences, wherein the nucleic acid encoding the antiangiogenic protein may contain those sequences necessary for expression of the sequence encoding the antiangiogenic protein and not partially or totally require these regulatory regions and/or sites of the virus genome. These regulatory sites may also be derived from another source, such a virus other than adenovirus or retrovirus. For example, and as described herein, a polyadenylation signal from SV40 may be used rather than an adenovirus, a human, or a murine polyadenylation signal. The sequence encoding the antiangiogenic protein may, alternatively, contain some sequences necessary for expression of the antiangiogenic protein and derive other sequences necessary for the expression of the antiangiogenic protein from the virus genome, or even from the host in which the recombinant virus is introduced.

As noted above, it is believed that, where desired, modification and changes may be made in the structure of the antiangiogenic protein and still obtain a molecule having like or otherwise desirable characteristics. Such changes may occur in natural isolates or may be synthetically introduced using site-specific mutagenesis, the procedures for which, such as mis-match PCR, are well known in the art.

For example, certain amino acids may be substituted for other amino acids in an antiangiogenic protein structure without appreciable loss of interactive binding capacity with structures such as antigen-binding regions of antibodies (or, e.g., binding sites on substrate or receptor molecules). Since it is the interactive capacity and nature of a protein that defines that protein's biological functional activity, certain amino acid sequence substitutions can be made in an antiangiogenic protein sequence (or, of course, its underlying nucleic acid sequence) and nevertheless obtain an antiangiogenic protein with like or even countervailing properties (e.g., antagonistic v. agonistic). It is thus contemplated by the inventors that various changes may be made in the sequence of the antiangiogenic protein (or underlying nucleic acid) without appreciable loss of their biological utility or activity and possibly with an increase in such utility or activity.

The nucleic acid encoding a antiangiogenic protein or a vector may also contain a selectable marker which can be used to screen for those cells which contain the nucleic acid or vector and which express the selectable marker. In this manner, one can readily separate those cells containing the nucleic acid or the vector and expressing the selectable marker from those cells either containing the nucleic acid or the vector but not expressing the selectable marker, and from those cells not containing the nucleic acid or the vector. The specific selectable marker used can of course be any selectable marker which can be used to select against eukaryotic cells not containing and expressing the selectable marker. The selection can be based on the death of cells not containing and expressing the selectable marker, such as where the selectable marker is a gene encoding a drug resistance protein. An example of such a drug resistance gene for eukaryotic cells is a neomycin resistance gene. Cells expressing a neomycin resistance gene are able to survive in the presence of the antibiotic G418, or Geneticin®, whereas those eukaryotic cells not containing or not expressing a neomycin resistance gene are selected against in the presence of G418. One skilled in the art will appreciate that there are other examples of selectable markers, such as the *hph* gene which can be selected for with the antibiotic Hygromycin B, or the *E. coli Ecogpt* gene which can be selected for with the antibiotic Mycophenolic acid. The specific selectable marker used is therefore variable.

The selectable marker can also be a marker that can be used to isolate those cells containing and expressing the selectable marker gene from those not containing and/or not expressing the selectable marker gene by a means other than the ability to grow in the presence of an antibiotic. For example, the selectable marker can encode a protein which, when expressed, allows those cells expressing the selectable marker encoding the marker to be identified. For example, the selectable marker can encode a luminescent protein, such as a luciferase protein or a green fluorescent protein, and the cells expressing the selectable marker encoding the luminescent protein can be identified from those cells not containing or not expressing the selectable marker encoding a luminescent protein. Alternatively, the selectable marker can be a sequence encoding a protein such as chloramphenicol acetyl transferase (CAT). By methods well known in the art, those cells producing CAT can readily be identified and distinguished from those cells not producing CAT.

The nucleic acid encoding an antiangiogenic protein can be a DNA, an RNA, or any combination thereof, whether containing only those bases typically found, or containing modified bases. These modified nucleotides are well known in the art and include, but are not limited to, thio-modified deoxynucleotide triphosphates and borano-modified deoxynucleotide triphosphates (Eckstein and Gish, *Trends in Biochem. Sci.,* 14:97-100 (1989) and Porter *Nucleic Acids Research,* 25:1611-1617 (1997)).

Alternatively, the nucleic acid can encode another type of signaling ligand and/or receptor such that when that ligand and/or receptor is introduced into a cell, and whereby the cell expresses the nucleic acid thereby producing the ligand and/or receptor, the interaction of the ligand and/or the receptor causes a tumor cell to undergo apoptosis, thereby treating the tumor cell and simultaneously expressing a nucleic acid sequence encoding the antiangiogenic protein. An example of other signal molecules that can be used in the methods of the present invention includes, but is not limited to, Bax, Bad, Bak, and Bik. (*Adams et al.* "Control of cell death" WEHI Annual Report 1996/1997).

In another embodiment of the present invention, the nucleic acid encoding the antiangiogenic protein can also encode another protein such as a regulatory protein, which may be used to regulate the expression of the antiangiogenic protein. For example, the regulatory protein can cause the tissue-specific localization of the antiangiogenic protein on the cell membrane, or alternatively cause the premature turnover of the antiangiogenic protein in non-target cells, or regulate the expression of the antiangiogenic protein via regulation of transcription and/or translation.

The regulatory protein can also be encoded by another nucleic acid that is delivered to the cell, either independently or consecutively. In this embodiment, the two nucleic acids can have different sequences, such as different promoters, such that they can be independently regulated, such as by the administration of a drug that selectively regulates the expression of one or both of the promoters, such as by the use of a steroid hormone, e.g. a glucocorticoid hormone that can regulate a promoter that is inducible by that hormone.

The nucleic acid encoding a antiangiogenic protein can also comprise a fusion protein. One skilled in the art will recognize that fusion proteins are routinely used for such purposes as localization of the protein, activation or deactivation of the protein, monitoring the location of the protein, isolation of the protein, and quantitating the amount of the protein. In one embodiment, the fusion protein comprises a antiangiogenic protein and a green fluorescent protein. Other examples of fusion proteins that comprise the antiangiogenic protein include the CAT gene, the neo gene, the hygromycin gene, and so forth.

The nucleic acid encoding a antiangiogenic protein can also contain a sequence that is capable of regulating the expression of the antiangiogenic protein. For example, the nucleic acid can contain a glucocorticoid regulatory element (GRE) such that glucocorticoid hormones can be used to regulate the expression of the antiangiogenic protein. Another example of a regulatory sequence that can regulate the expression of an adjacent gene is by cloning an RNA aptamer, such as H10 and H19, into the promoter region whereby administration of a drug such as Hoechst dye 33258 can block expression of the gene in vivo. (*Werstuck et al.* "Controlling gene expression in living cells through small molecule-RNA interactions" Science 282:296-298 (1998)). In other embodiments of the present invention, the regulatory sequence comprises the Tet-operon or the lac operon, or any other operon that can function as a regulatory sequence in a eukaryotic cell.

The methods described herein comprise introducing into a cell a nucleic acid encoding an antiangiogenic protein. One skilled in the art will recognize that this aspect of the methods can comprise either a stable or a transient introduction of the nucleic acid construct into the cell. Additionally, the stably or the transiently introduced nucleic acid may or may not become integrated into the genome of the host. One skilled in the art will also recognize that the precise procedure for introducing the nucleic acid into the cell may, of course, vary and may depend on the specific type or identity of the cell. Examples of methods for introducing a nucleic acid into a cell include, but are not limited to electroporation, cell fusion, DEAE-dextran mediated transfection, calcium phosphate-mediated transfection, infection with a viral vector, microinjection, lipofectin-mediated transfection, liposome delivery, and particle bombardment techniques such as gene delivery on gold particles, including various procedures for "naked DNA" delivery.

In one embodiment of the present invention, the promoter is a tissue-specific promoter which one skilled in the art will appreciate can confer tissue-specificity to the expression of the nucleic acid encoding the antiangiogenic protein. For example, the tissue-specific promoter may be a prostate-specific, a breast tissue-specific, a colon tissue-specific, a brain-specific, a kidney-specific, a liver-specific, a bladder-specific, a lung-specific, a thyroid-specific a stomach-specific, a ovary-specific, or a cervix-specific promoter. Where the tissue-specific promoter is a prostate-specific promoter, the promoter includes, but is not limited to the PSA promoter, the ΔPSA promoter, the ARR2PB promoter, and the PB promoter.

The tissue-specificity can also be achieved by selecting a vector that has a high degree of tissue specificity. For example, a vector that selectively infects mucosal cells, such as those associated with colon cancer, can be chosen, and then optionally, used in combination with a specific delivery means, such as by the use of a suppository, to selectively deliver the nucleic acid encoding antiangiogenic protein to those desired cells.

The various vectors and hosts used to express the nucleic acid encoding a antiangiogenic protein may be used to express the nucleic acids in culture or *in vitro.* For example, a vector comprising a nucleic acid encoding a antiangiogenic protein may be introduced into a tissue culture cell line, such as COS cells, and expressed whereby the nucleic acid is expressed in culture. In this manner, one skilled in the art can select a cell type that may have a limited life in the host organism such that the host can effectively clear the cell expressing the antiangiogenic protein in a period of time such that any possible deleterious effects on non-target surrounding cells or tissues can be minimized. Alternatively, cells from a subject may be removed from the subject, administered the nucleic acid encoding a antiangiogenic protein, and then replaced into the subject. In this *ex vivo* treatment procedure, the cells can be manipulated to facilitate the uptake of the nucleic acid encoding a antiangiogenic protein without unnecessary adverse effects on the subject.

Alternatively, various vectors and hosts used to express the nucleic acids of the present invention may be used to express the nucleic acids *in vivo.* For example, a vector comprising a nucleic acid encoding an antiangiogenic protein may be introduced into cells of a eukaryotic host, preferably tumor cells, to treat tumor cells *in situ.* As briefly discussed above, one skilled in the art will appreciate that specific tissues can be treated by selectively administering the vector to the host. For example, administering an adenovirus vector via an aerosol such as through the use of an inhaler can selectively administer the vector to the lungs. Alternatively, the use of a suppository can be used to selectively administer the vector to cells of the colon. Alternatively, delivering the vector topically such as in a cream can selectively deliver the vector or nucleic acid to skin cells or the cervix. One skilled in the art will recognize the various methods that can routinely be used to selectively deliver the vector, or alternatively, the nucleic acid encoding a antiangiogenic protein, to specific organs or cells.

One skilled in the art will also appreciate that delivery can be manually facilitated through such methods as injection of the vector or the nucleic acid to the selected site. For example, direct injection can be used to deliver the vector or nucleic acid to specific brain and/or breast location.

It is contemplated that using the methods of the present invention, a nucleic acid encoding antiangiogenic protein can be administered to a cell or to a subject, most preferably, humans, to treat disease states, preferably those comprising tumor development. The methods of the present invention can also be used to treat any disease or process mediated by angiogenesis including, but not limited to leukemia, metastasis, rheumatoid diseases, diabetic neovascularization, hematopoiesis, and wound healing. The present nucleic acid, whether alone, in combination with another compound or composition, or as part of a vector-based delivery system, may be administered parenterally (e.g., intravenously), by intramuscular injection, by intraperitoneal injection, topically, transdermally, or the like, although topical administration is typically preferred. The exact amount of such nucleic acids, compositions, vectors, etc., required will vary from subject to subject, depending on the species, age, weight and general condition of the subject, the severity of the disease or condition that is being treated, the particular compound or composition used, its mode of administration, and the like. Thus, it is not possible or necessary to specify an exact amount. However, an appropriate amount may be determined by one of ordinary skill in the art using methods well known in the art (see, e.g., *Martin et al.,* 1989).

For topical administration, the virus or nucleic acid encoding antiangiogenic protein, compositions thereof, and/or vectors comprising the nucleic acid can be in pharmaceutical compositions in the form of solid, semi-solid or liquid dosage forms, such as, for example powders, liquids, suspension, lotions, creams, gels or the like, preferably in unit dosage form suitable for single administration of a precise dosage. The compositions can typically include an effective amount of the selected nucleic acid, composition, or vector in combination with a pharmaceutically acceptable carrier and, in addition, may include other medicinal agents, pharmaceutical agents, carriers, adjuvants, diluents, etc. By "pharmaceutically acceptable" is meant a material that is not biologically or otherwise undesirable, i.e., the material may be administered to an individual along with the selected nucleic acid, composition thereof, or vector without causing any undesirable biological effects or interacting in a deleterious manner with any of the other components of the pharmaceutical composition in which it is contained.

Alternatively or additionally, parenteral administration, if used, is generally characterized by injection e.g., by intravenous injection including regional perfusion through a blood vessel supplying the tissues(s) or organ(s) having the target cell(s). Injectables can be prepared in conventional forms, either as liquid solutions or suspensions, solid forms suitable for solution or suspension in liquid prior to injection, or as emulsions. Parenteral administration can also employ the use of a slow release or sustained release system, such that a constant level of dosage is maintained (See, for example, U.S. Patent No. 3,710,795). The compound can be injected directly to the site of cells or tissues comprising a tumor, or they can be injected such that they diffuse or circulate to the site of the tumor cells.

Dosages will depend upon the mode of administration, the disease or condition to be treated, and the individual subject's condition. Dosages will also depend upon the material being administered, e.g., a nucleic acid, a vector comprising a nucleic acid, or another type of compound or composition. Such dosages are known in the art. Furthermore, the dosage can be adjusted according to the typical dosage for the specific disease or condition to be treated. Furthermore, culture cells of the target cell type can be used to optimize the dosage for the target cells *in vivo,* and transformation from varying dosages achieved in culture cells of the same type as the target cell type can be monitored. Often a single dose can be sufficient; however, the dose can be repeated if desirable. The dosage should not be so large as to cause adverse side effects. Generally, the dosage will vary with the age, condition, sex and extent of the disease in the patient and can be determined by one of skill in the art. The dosage can also be adjusted by the individual physician in the event of any complication.

For administration to a cell in a subject, the compound or composition, once in the subject, will of course adjust to the subject's body temperature. For *ex vivo* administration, the compound or composition can be administered by any standard methods that would maintain viability of the cells, such as by adding it to culture medium (appropriate for the target cells) and adding this medium directly to the cells. As is known in the art, any medium used in this method can be aqueous and non-toxic so as not to render the cells non-viable. In addition, it can contain standard nutrients for maintaining viability of cells, if desired. For *in vivo* administration, the complex can be added to, for example, a blood sample or a tissue sample from the patient, or to a pharmaceutically acceptable carrier, e.g., saline and buffered saline, and administered by any of several means known in the art. Other examples of administration include inhalation of an aerosol, subcutaneous or intramuscular injection, direct transfection of a nucleic acid sequence encoding the compound where the compound is a nucleic acid or a protein into, e.g., bone marrow cells prepared for transplantation and subsequent transplantation into the subject, and direct transfection into an organ that is subsequently transplanted into the subject. Further administration methods include oral administration, particularly when the composition is encapsulated, or rectal administration, particularly when the composition is in suppository form. A pharmaceutically acceptable carrier includes any material that is not biologically or otherwise undesirable, i.e., the material may be administered to an individual along with the selected complex without causing any undesirable biological effects or interacting in a deleterious manner with any of the other components of the pharmaceutical composition in which it is contained.

Specifically, if a particular cell type *in vivo* is to be targeted, for example, by regional perfusion of an organ or tumor, cells from the target tissue can be biopsied and optimal dosages for import of the complex into that tissue can be determined *in vitro,* as described herein and as known in the art, to optimize the *in vivo* dosage, including concentration and time length. Alternatively, culture cells of the same cell type can also be used to optimize the dosage for the target cells *in vivo.* For example, intratumoral injection amounts and rates can be controlled using a controllable pump, such as a computer controlled pump or a micro-thermal pump, to control the rate and distribution of the nucleic acid or vector in the tumor or tissue.

For either *ex vivo, in vivo, in vitro,* or in culture use, the nucleic acid, vector, or composition can be administered at any effective concentration. An effective concentration is that amount that results in partial or total killing, reduction in size, disappearance, inhibition of growth, inhibition of vascularization, inhibition of cellular proliferation, an induction in dormancy or an apparent induction of dormancy, or a decreased metastasis of a tumor or a tumor cell. These mechanisms of action are only exemplary of the ways an antiangiogenic protein can treat a tumor, and the primary affect appears to be through a decrease in the vascularization of a tumor.

The nucleic acid or vector can be administered in a composition. For example, the composition can comprise other medicinal agents, pharmaceutical agents, carriers, adjuvants, diluents, etc. Furthermore, the composition can comprise, in addition to the nucleic acid or vector, lipids such as liposomes, such as cationic liposomes (e.g., DOTMA, DOPE, DC-cholesterol) or anionic liposomes. Liposomes can further comprise proteins to facilitate targeting a particular cell, if desired. Administration of a composition comprising a nucleic acid or a vector and a cationic liposome can be administered to the blood afferent to a target organ or inhaled into the respiratory tract to target cells of the respiratory tract. Regarding liposomes, see, e.g., Brigham et al. *Am. J. Resp. Cell. Mol. Biol.* 1:95-100 (1989); Felgner et al. *Proc. Natl. Acad. Sci USA* 84:7413-7417 (1987); U.S. Pat. No.4,897,355. Furthermore, the nucleic acid or a vector can be administered as a component of a microcapsule that can be targeted to specific cell types, such as macrophages, or where the diffusion of the compound or delivery of the compound from the microcapsule is designed for a specific rate or dosage.

Any cell, preferably a cell near or within a tumor, can be induced used in the methods of the present invention. In this embodiment, these cells can selectively be administered a nucleic acid encoding and expressing an antiangiogenic protein, thereby treating a tumor by any one or more of the mechanisms listed above. In one embodiment, the tumor can be a solid tumor and the tumor or adjacent tissue is injected with a recombinant virus which can infect the cells and thereby cause them to express an antiangiogenic protein, and whereby the presence of an antiangiogenic protein treats the tumor. The cells which are affected by the antiangiogenic protein are typically cells adjacent to the antiangiogenic protein-expressing cells since preferably the antiangiogenic protein is secreted from the cells expressing the nucleic acid encoding the antiangiogenic protein. The affected cell can be removed from the immediate surroundings of the antiangiogenic protein-expressing cell, however, such as where the antiangiogenic protein-expressing cell has mobilized and/or where the antiangiogenic protein-expressing cell produces soluble antiangiogenic protein that is circulated. The antiangiogenic protein-expressing cells can also cause their own death since antiangiogenic protein can also affect those cells. In this approach, the methods of the present invention can cause cells expressing an antiangiogenic protein to die, but adjacent tumor cells will also die, thereby eliminating those tumor cells that might otherwise cause regression of the tumor.

The present invention also provides an adenovirus comprising a recombinant nucleic acid encoding an antiangiogenic protein inserted within an adenoviral nucleic acid, wherein the recombinant nucleic acid can be packaged in an adenovirus particle and wherein expression of the nucleic acid encoding the antiangiogenic protein results in production of the antiangiogenic protein. Various adenoviruses may be used in the compounds and methods described herein. For example, and as described in the Example contained herein, a nucleic acid encoding an antiangiogenic protein can be inserted within the genome of adenovirus type 5. Similarly, other types of adenovirus may be used such as type 1, type 2, type 3, etc. For an exemplary list of the adenoviruses known to be able to infect human cells and which therefore can be used in the present invention, see *Fields, et al.* (1990) Virology, Raven Press, New York). Furthermore, it is contemplated that a recombinant nucleic acid comprising an adenoviral nucleic acid from one type adenovirus can be packaged using capsid proteins from a different type adenovirus.

The virus can be one that is replication deficient, depending upon the specific application of the compounds and methods described herein. Methods of rendering a virus replication deficient and are well known in the art. For example, mutations such as point mutations, deletions, and insertions, and combinations thereof, can be directed toward a specific adenoviral gene or genes, such as the E1 gene. For a specific example of the generation of a replication deficient adenovirus for use in gene therapy, see WO 94/28938 (Adenovirus Vectors for Gene Therapy Sponsorship).

In all the constructions or compounds described herein, the antiangiogenic protein may be functionally attached to a specific leader peptide which can specify for secretion of the protein. For example and as described in the Example below, the antiangiogenic protein can have a signal sequence, such as the adenovirus E3/K19 signal sequence that can result in secretion of the antiangiogenic protein from the cell which is expressing the sequence encoding the antiangiogenic protein. A specific example of an adenovirus E3/k19 signal sequence comprises the amino acid sequence MRYMILGLLALAAVCSAA. A functional attachment is typically, but not limited to, a peptide bond. Other additional sequences may also be attached to the antiangiogenic protein, either through the addition of a nucleic acid encoding the additional sequence, or by addition of a peptide to the antiangiogenic protein. Similarly, the specific antiangiogenic proteins of the present invention may be obtained not only through expression of a nucleic acid, but through the synthesis of a polypeptide as well. One skilled in the art will recognize that different nucleic acid sequences can encode the same polypeptide and therefore the exact sequence of the nucleic acid encoding the signal sequence can vary.

One method of producing proteins comprising the antiangiogenic proteins of the present invention is to link two or more peptides or polypeptides together by protein chemistry techniques. For example, peptides or polypeptides can be chemically synthesized using currently available laboratory equipment using either Fmoc (9-fluorenylmethyloxycarbonyl) or Boc (*tert*-butyloxycarbonoyl) chemistry. (Applied Biosystems, Inc., Foster City, CA). One skilled in the art can readily appreciate that a peptide or polypeptide corresponding to endostatin, for example, can be synthesized by standard chemical reactions. For example, a peptide or polypeptide can be synthesized and not cleaved from its synthesis resin whereas the other fragment of an antiangiogenic protein can be synthesized and subsequently cleaved from the resin, thereby exposing a terminal group which is functionally blocked on the other fragment. By peptide condensation reactions, these two fragments can be covalently joined via a peptide bond at their carboxyl and amino termini, respectively, to form an antiangiogenic protein. (*Grant, G.A.,* "Synthetic Peptides: A User Guide" W.H. Freeman and Co., N.Y. (1992) and *Bodansky, M. and Trost, B.,* Ed., "Principles of Peptide Synthesis" Springer-Verlag Inc., N.Y. (1993)). Alternatively, the peptide or polypeptide can by independently synthesized *in vivo* as described above. Once isolated, these independent peptides or polypeptides may be linked to form an antiangiogenic protein via similar peptide condensation reactions.

For example, enzymatic ligation of cloned or synthetic peptide segments can allow relatively short peptide fragments to be joined to produce larger peptide fragments, polypeptides or whole protein domains (*Abrahmsen, L., el al.,* Biochemistry, 30:4151 (1991)). Alternatively, native chemical ligation of synthetic peptides can be utilized to synthetically construct large peptides or polypeptides from shorter peptide fragments. This method consists of a two step chemical reaction (*Dawson, et al.,* "Synthesis of Proteins by Native Chemical Ligation" *Science,* 266:776-779 (1994)). The first step is the chemoselective reaction of an unprotected synthetic peptide-%-thioester with another unprotected peptide segment containing an amino-terminal Cys residue to give a thioester-linked intermediate as the initial covalent product. Without a change in the reaction conditions, this intermediate undergoes spontaneous, rapid intramolecular reaction to form a native peptide bond at the ligation site. Application of this native chemical ligation method to the total synthesis of a protein molecule is illustrated by the preparation of human interleukin 8 (IL-8) (*Clark-Lewis, I., et al.,* FEBS Lett., 307:97 (1987), *Clark-Lewis, L, et al.,* J.Biol.Chem., 269:16075 (1994), *Clark-Lewis, I., et al.,* Biochemistry, 30: 3128 (1991), and *Rajarathnam, K., et al.,* Biochemistry, 29:1689 (1994)).

Alternatively, unprotected peptide segments can be chemically linked where the bond formed between the peptide segments as a result of the chemical ligation is an unnatural (non-peptide) bond (*Schnolzer, M., et al.,* Science, 256:221 (1992)). This technique has been used to synthesize analogs of protein domains as well as large amounts of relatively pure proteins with full biological activity (*deLisle Milton, R.C., et al.,* "Techniques in Protein Chemistry IV" Academic Press, New York, pp. 257-267 (1992)).

The invention also discloses fragments of antiangiogenic proteins which have bioactivity. The polypeptide fragments of the present invention can be recombinant proteins obtained by cloning nucleic acids encoding the polypeptide in an expression system capable of producing the polypeptide fragments thereof, such as the adenovirus system described herein. For example, one can determine the active domain of endostatin which can cause a biological effect associated with the endostatin. In one example, amino acids found to not contribute to either the activity or the binding specificity or affinity of the endostatin can be deleted without a loss in the respective activity.

For example, amino or carboxy-terminal amino acids can be sequentially removed from an antiangiogenic protein molecule and the respective activity assayed in one of many available assays. In another example, a fragment of an antiangiogenic protein can comprise a modified endostatin wherein at least one amino acid has been substituted for the naturally occurring amino acid at a specific position, and a portion of either amino terminal or carboxy terminal amino acids, or even an internal region of the endostatin, has been replaced with a polypeptide fragment or other moiety, such as biotin, which can facilitate in the purification of the modified antiangiogenic protein. For example, a modified antiangiogenic protein can be fused to a maltose binding protein, through either peptide chemistry of cloning the respective nucleic acids encoding the two polypeptide fragments into an expression vector such that the expression of the coding region results in a hybrid polypeptide. The hybrid polypeptide can be affinity purified by passing it over an amylose affinity column, and the modified antiangiogenic protein receptor can then be separated from the maltose binding region by cleaving the hybrid polypeptide with the specific protease factor Xa. (See, for example, New England Biolabs Product Catalog, 1996, pg. 164.). Similar purification procedures are available for isolating hybrid proteins from eukaryotic cells as well.

Active fragments of an antiangiogenic protein can also be synthesized directly or obtained by chemical or mechanical disruption of a larger antiangiogenic protein. An active fragment is defined as an amino acid sequence of at least about 5 consecutive amino acids derived from the naturally occurring amino acid sequence, which has the relevant activity, e.g., binding or regulatory activity.

The fragments, whether attached to other sequences or not, can also include insertions, deletions, substitutions, or other selected modifications of particular regions or specific amino acids residues, provided the activity of the peptide is not significantly altered or impaired compared to the nonmodified antiangiogenic protein or antiangiogenic protein fragment. These modifications can provide for some additional property, such as to remove/add amino acids capable of disulfide bonding, to increase its bio-longevity, to alter its secretory characteristics, etc. In any case, the peptide must possess a bioactive property, such as binding activity, regulation of binding at the binding domain, etc. Functional or active regions of the antiangiogenic protein may be identified by mutagenesis of a specific region of the protein, followed by expression and testing of the expressed polypeptide. Such methods are readily apparent to a skilled practitioner in the art and can include site-specific mutagenesis of the nucleic acid encoding the receptor. (*Zoller, M.J. et al.*).

The present invention also discloses a method of delivering an antiangiogenic protein to a cell comprising administering to the cell a virus comprising a recombinant nucleic acid encoding an antiangiogenic protein inserted within the viral nucleic acid, wherein the recombinant nucleic acid can be packaged in a virus particle, and wherein expression of the recombinant nucleic acid encoding the antiangiogenic protein results in production of the antiangiogenic protein, thereby delivering the antiangiogenic protein to the cell. Such administration can result in highly successful delivery of the nucleic acid to cells and thus relatively high levels of expression of the sequence encoding the antiangiogenic protein.

It is well known in the art that an adenovirus can infect a wide variety of cells and therefore deliver its nucleic acid to the host cell, which in turn, can then express the nucleic acid, thereby producing the proteins encoded by the adenoviral nucleic acid. For example, adenovirus can infect various sites of the respiratory tract, the eye, muscle cells, cells of the gastrointestinal tract, and cells of the bladder. As discussed above, the cell to which the adenovirus comprising a nucleic acid encoding an antiangiogenic protein is administered may comprise a cell *ex vivo,* such as a cell removed from a subject which is administered the adenovirus and subsequently replaced back to the subject. Alternatively, the cell may comprise a cell *in vivo,* such as delivering the adenovirus comprising the recombinant nucleic acid encoding an antiangiogenic protein to a cell within or on a subject. Alternatively, the cell may comprise a cell *in culture,* such as delivering to tissue culture cells an adenovirus comprising a sequence encoding the antiangiogenic protein.

This method, therefore, can be used to deliver an antiangiogenic protein to a particular cell or a group of cells, or alternatively, to a particular tissue or organ. Once an adenovirus comprising a sequence encoding the antiangiogenic protein or a recombinant nucleic acid encoding an antiangiogenic protein is administered to a cell, that cell can then express the recombinant nucleic acid encoding the antiangiogenic protein and thereby produce the antiangiogenic protein, which can have a biological response from that cell or other cells which contact the antiangiogenic protein or are otherwise affected by the antiangiogenic protein.

The invention also discloses a method of delivering an antiangiogenic protein to a subject comprising administering to the subject a virus comprising a recombinant nucleic acid encoding an antiangiogenic protein inserted within the viral nucleic acid, wherein the recombinant nucleic acid can be packaged in a virus particle, and whereby a cell of the subject expresses the recombinant nucleic acid encoding the antiangiogenic protein, thereby delivering the antiangiogenic protein to the subject.

A compound comprising an adenovirus comprising a nucleic acid encoding an antiangiogenic protein, a recombinant nucleic acid encoding an antiangiogenic protein, or a virus comprising a nucleic acid encoding an antiangiogenic protein of this invention can be administered to a subject in need thereof by commonly employed methods for administering compounds in such a way to bring the adenovirus and/or the antiangiogenic protein in contact with the tissue or cell to be treated. Such methods include oral administration, parenteral injection (IP), subcutaneous injection (SC -particularly a controlled release depot), intravenous injection (IV), intramuscularly (IM), intranasal (IN), intraocular (IO), extraocular (EO), sublingual (SL), or orally such as through oral inhalation (OI). In general, a therapeutically effective amount is that amount needed to achieve the desired results, thus successfully treating of the targeted disease state. Articles by George Hiller, *Advanced Drug-Delivery Reviews,* 10: 163-204 (1993) [Elsevier Science Publishers B.V.] and Lorraine L. Wearley, *Critical Reviews in Therapeutic Drug Carrier Systems,* 8(4): 331-394 (1991) are useful discussions of these types of administrations. These articles are incorporated herein by reference.

The administration of the compounds of this invention can be combined with other therapies such as surgery, chemotherapy, radiotherapy, immunotherapy or any combination thereof. Examples of chemotherapeutic agents include cisplatin, 5-fluorouracil and S-1. Immunotherapeutic methods include administration of interleukin-2 and interferon-$\alpha$.

The dosage ranges for the administration of a compound or compositions of this invention may depend upon its potency, as described further herein, and are amounts large enough to produce the desired effect in which the condition being treated is treated, e.g. is measurably prevented, inhibited or decreased. The dosage should not be so large as to cause adverse side effects. Generally, the dosage will vary with the age, condition, sex and extent of the condition in the subject and can be determined by one of skill in the art. The dosage can also be adjusted by the individual physician in the event of any complication. The dosage can be that amount typical for related adenovirus administrations, such as about $1 \times 10^2$ to about $1 \times 10^{12}$ plaque-forming units of adenovirus and can be, for example, $1 \times 10^2$ pfu, $1 \times 10^3$ pfu, $1 \times 10^4$ pfu, $1 \times 10^5$ pfu, $1 \times 10^6$ pfu, $1 \times 10^7$ pfu, $1 \times 10^8$ pfu, $1 \times 10^9$ pfu, $1 \times 10^{10}$ pfu, $1 \times 10^{11}$ pfu, $1 \times 10^{12}$ pfu. For administration of recombinant virus comprising a nucleic acid encoding an antiangiogenic protein, the dose can range from about .001 to about 10 mg/kg and can be, for example, about 0.01 to about 10 mg/kg; about 0.1 to about 10mg/k; about 1.0 to about 10 mg/kg; about 0.001 to about 1 mg/kg; about 0.001 to about 0.1 mg/kg; and about 0.01 to about 1.0 mg/kg. For one trial the dose used was up to I mg/kg (See, *Hodges et al.* "Phase I Study of Recombinant Human CD4-Immunoglobulin G Therapy of Patients with AIDS and AIDS-related Complex" Antimicrob. Agents Chemother. 35:2580-6 (1991).

The therapeutic compounds or compositions of this invention are conventionally administered, whether by IP, IM, IV, IN, IO, EO, SubCu, etc., as of a unit dose. The term "unit dose" when used in reference to a therapeutic compound or composition of the present invention refers to physically discrete units suitable as unitary dosage for the subject, each unit containing a predetermined quantity of active material calculated to produce the desired therapeutic effect in association with the required diluent; i.e., carrier, or vehicle.

The compounds or compositions are administered in a manner compatible with the dosage formulation, and in a therapeutically effective amount. The quantity to be administered depends on the subject to be treated, capacity of the subject's system to utilize the active ingredient, and degree of therapeutic effect desired. Precise amounts of active ingredient required to be administered depend on the judgement of the practitioner and are peculiar to each individual. However, suitable dosage ranges for systemic application may depend on the route of administration. Suitable regimes for administration are also variable, but are typified by an initial administration followed by repeated doses at one or more hour intervals by a subsequent injection or other administration. Alternatively, continuous intravenous infusion sufficient to maintain concentrations in the blood in the ranges specified for *in vivo* therapies are contemplated.

Another aspect of this invention is a pharmaceutically-acceptable therapeutic composition that comprises a therapeutically effective amount of a virus or an antiangiogenic protein of this invention in combination with a pharmaceutically-acceptable excipient. The composition is designed to facilitate the method of administering a virus or an antiangiogenic protein of this invention in an effective manner. Generally a composition of this invention will have a virus or an antiangiogenic protein dissolved or dispersed in the pharmaceutically-acceptable excipient.

As used herein, the terms "pharmaceutically acceptable", "physiologically tolerable" and grammatical variations thereof, as they refer to compositions, excipients (including carriers, diluents, stabilizers, lubricants, reagents and the like), are used interchangeably and represent that the materials are capable of administration to or upon a subject without toxicity and preferably without the production of undesirable physiological effects such as nausca, dizziness, gastric upset and the like.

Compounds or compositions of this invention may be administered to a subject in a variety of forms depending on the method of administration. The method of administration may be viewed as "invasive" (e.g., IV, IM, IP or SC) or "non-invasive" (e.g., ocular, buccal, oral, transdermal, rectal, NI, OI [pulmonary], and the like). In general, a composition that is delivered by an invasive route is generally administered by a health care professional while a composition delivered by a non-invasive route may be administered by the patient him-or herself.

In administering the compounds or compositions of the invention by non-invasive method there are various general methods that are used for enhancing the delivery of a compound comprising an adenovirus containing a nucleic acid encoding an antiangiogenic protein, a nucleic acid encoding an adenovirus encoding an antiangiogenic protein, or an antiangiogenic protein. The first is to increase the absorption of the compound. This can be done by the use of a prodrug, chemical modification of the primary structure of the compound, incorporation of the compound into liposomes or other encapsulation material, co-administration with penetration enhancers, the use of physical methods such as iontophoresis and phonophoresis and targeting to specific tissues. Another method for enhancing delivery is to minimize the metabolism of the compound. This would include chemical modification of the primary structure, covalent attachment to a polymer, incorporation into a liposome or other encapsulation material, co-administration with an enzyme inhibitor and targeting to specific tissues. The third general method of enhancing delivery of the compound includes prolonging the half-life of the compound by protecting it with polymers or liposomes, using a bioadhesive material or targeting the composition to a specific tissue.

In general, if it is desired to increase the absorption of the compounds of this invention through ocular, buccal, transdermal, or rectal administration, or by nasal inhalation or oral inhalation, one can employ certain penetration enhancers. These enhancers can include chelators such as EDTA, citric acid, N-acyl derivatives of collagen, enamines (N-Amino N-acyl derivatives of diketones). Surfactants can also be used to enhance penetration. These include, sodium lauryl sulfate, polyoxyethylene-9-lauryl ether and polyoxyethelene-20-cetyl ether. Bile salts and derivatives are also known to enhance the penetration of the compound and these include sodium deoxycholate, sodium glycocholate, sodium taurocholate, sodium taurodihydrofusidate and sodium glycodihyrofusidate. Still another type of penetration enhancer useful in the composition of this invention includes ceratin fatty acids and derivatives such as oliec, caprylic acid, capric acid, acylcarnitines, acylcholine and mono and diglycerides. Nonsurfactants are also useful as penetration enhancers. The penetration enhancers can be used in the solution with the compounds of this invention where the compound and the penetration enhancers are in a pharmaceutically acceptable sterile solution which can be administered, for example by ocular administration. Alternatively the penetration enhancers can be included in a powdered formulation that can be administered as a aerosol by suspending the particulate matter in the stream of air. Such powdered formulations can be administered by a dry-powder inhaler such as those represented by Ventolin Rotohaler (Glaxo, Inc., Research Triangle Park, North Carolina, U.S.A), and Spinhaler (Fisons Corporation, Bedford, MA). Compositions that are in the form of solid micronized particle having a particle size of about 0.5 to 10 microns in median diameter may be prepared in accordance with the teaching of PCT application international publication numbers WO91/16038 and WO93/00951. Powders may be prepared in accordance with the teaching of Remington's Pharmaceutical Sciences, 18th Edition, Mack Publishing Co., Chapter 88: 1645-1648. These teachings are incorporated herein by reference.

The active compound may be orally administered, for example, with an inert diluent or with an assimilable edible carrier, or it may be enclosed in hard or soft shell gelatin capsules, or it may be compressed into tablets, or it may be incorporated directly with the food of the diet. For oral therapeutic administration, the active compound may be incorporated with excipient and used in the form of ingestible tablets, buccal tablets, troches, capsules, elixirs, suspensions, syrups, wafers, and the like. Such compositions and preparations should contain at least 0.1 % of active compound. In preparing oral formulations one needs to be aware of the problems of degradation in the mouth and upper GI tract. Thus, it may be preferable to employ an enzyme inhibitor in combination with the compound, or to use a penetration enhancer or to use a protective polymer or microcapsule. The percentage of the compositions and preparations may, of course, be varied and may conveniently contain up to about 20% by weight of the compound in a dosage unit. The amount of active compound in such therapeutically useful compositions is such that a suitable dosage will be obtained.

The tablets, troches, pills, capsules and the like may also contain excipients such as the following: a binder such as polyvinylpyrrolidone, gum tragacanth, acacia, sucrose, com starch, gelatin, calcium phosphate, sodium citrate, and calcium carbonate; a disintegrant such as com starch, potato starch, tapioca starch, certain complex silicates, alginic acid and the like; a lubricant such as sodium lauryl sulfate, talc and magnesium stearate; a sweetening agent such as sucrose, lactose or saccharin; or a flavoring agent such as peppermint, oil of wintergreen or cherry flavoring. Solid compositions of a similar type are also employed as fillers in soft and hard-filled gelatin capsules; materials in this connection also include lactose or milk sugar as well as high molecular weight polyethylene glycols. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye, flavoring such as cherry or orange flavor, emulsifying agents and/or suspending agents, as well as such diluents as water, ethanol, propylene glycol, glycerin and various like combinations thereof. Of course, any material used in preparing any dosage unit form should be pharmaceutically pure and substantially non-toxic in the amounts employed. In addition, the active compound may be incorporated into sustained-release preparations and formulations. Further components may be apparent to one of ordinary skill in the art.

For purposes of IP administration, solutions in sesame or peanut oil or in aqueous propylene glycol can be employed, as well as sterile aqueous solutions of the corresponding water-soluble, alkali metal or alkaline-earth metal salts. Such aqueous solutions should be suitably buffered and the liquid diluent first rendered isotonic with sufficient saline or glucose. Solutions of the active compound as a free base or a pharmacologically acceptable salt can be prepared in water suitably mixed with e.g. hydroxypropylcellulose. A dispersion can also be prepared in glycerol, liquid polyethylene glycols and mixtures thereof and in oils. Under ordinary conditions of storage and use, these preparations contain a preservative to prevent the growth of microorganisms. These particular aqueous solutions are especially suitable for IV, IM, SC and IP. In this connection, the sterile aqueous media employed are all readily obtainable by standard techniques well-known to those skilled in the art.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, liquid polyethylene glycol and the like), suitable mixtures thereof, and vegetable oils. The proper fluidity can be maintained, for example, by the use of a coating such as lecithin, by the maintenance of the required particle size in the case of a dispersion and by the use of surfactants. The prevention of the action of microorganisms can be brought about by various antibacterial and antifungal agents, for example, parabens, chlorobutanol, phenol, sorbic acid, thimerosal and the like. In many cases it will be preferable to include isotonic agents, for example, sugars or sodium chloride. Prolonged absorption of the injectable compositions can be brought about by use of agents delaying absorption, for example, aluminum monostearate and gelatin.

Sterile injectable solutions are prepared by incorporating the active compound in the required amount in the appropriate solvent with various of the other ingredients enumerated above, and as required and appropriate, followed by filtered sterilization. Generally, dispersions are prepared by incorporating the sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze drying technique which yield a powder of the active ingredient plus any additional desired ingredient from the previously sterile-filtered solution thereof.

For IP formulations that are controlled-release, a compound of this invention can be combined with a polymer that regulates the release of the compound and protects it from degradation. Generally such polymer may be biodegradable or non-biodegradable and may further be hydrophilic or hydrophobic. Suitable hydrophilic, non-degradable polymers for use in the composition of this invention include hydrogels such as acrylamide or vinyl pyrrolidone crosslinked with N, N'-methylene bisacrylamide. Suitable non-degradable hydrophobic polymers include, ethylene/vinyl acetate copolymers, silicone elastomers, polydimethylsiloxane, and the like. Degradable hydrophilic polymers useful in this invention include N-vinyl pyrrolidone or acrylamide crosslinked with less than 1% N, N'-methylene bisacrylamide, dextran derivatized with glycidyl methacrylate and crosslinked with N, N'-methylene bisacrylamide, water-soluble polyester prepared from fumaric acid and poly (ethylene glycol) and crosslinked with N-vinyl pyrrolidone, water-soluble polyesters, and the like. Suitable degradable hydrophobic polymers useful for the composition of this invention include lactide/glycolide co-polymers, poly (orthoesters) and polyanhydrides. Of these various polymers, the lactide/glycolide co-polymers are preferred. A more detailed description of these polymers for controlled parenteral delivery may be found in an article by George Heller, *Advanced Drug-Delivery Reviews,* 10:163-204 (1993) (Elsevier Science Publishers BV). The article is incorporated herein by reference.

For the preferred controlled release composition of this invention the lactide/glycolide co-polymers may have a ratio of DL-a lactic acid to DL-glycolic acid of about 30:70 to about 70:30, preferably about 40:60 to about 60:40. A ratio of about 44:56 is representative. An adenovirus or antiangiogenic protein of this invention can be microencapsulated in the copolymer by means known in the art to form the composition, see, for example, U.S. Patent No. 4,675,189 issued June 23, 1987 to Sanders, Kent, Lewis and Tice.

For purposes of topical administration, dilute sterile, aqueous solutions (usually in about 0.1% to 5% concentration), otherwise similar to the above parenteral solutions, are prepared in containers suitable for drop-wise administration to the eye in appropriate vehicles such as saline with appropriate antimicrobial agents such as sodium benzoate and thimerasol.

The dosage of the present therapeutic agents which will be most suitable for prophylaxis or treatment will vary with the form of administration, the particular compound chosen and the physiological characteristics of the particular subject under treatment. Generally, small dosages may be used initially and, if necessary, increased by small increments until the optimum effect under the circumstances is reached. Oral administration may require higher dosages.

The present invention also discloses a method of treating a tumor in a subject comprising administering to the subject a virus comprising a recombinant nucleic acid encoding an antiangiogenic protein inserted within the viral nucleic acid, wherein the recombinant nucleic acid can be packaged in a virus particle, and whereby a cell in the subject expresses the recombinant nucleic acid encoding the antiangiogenic protein and produces the antiangiogenic, thereby treating the tumor.

As previously discussed, the recombinant nucleic acid encoding the antiangiogenic protein may be replication competent or replication deficient. This method therefore provides a method for treating a tumor in a subject by administering a recombinant nucleic acid encoding an antiangiogenic protein to the subject whereby the subject expresses the recombinant nucleic acid encoding the antiangiogenic protein thereby producing the antiangiogenic protein which treats the tumor. Also as discussed above, methods of administering the recombinant nucleic acid encoding the antiangiogenic protein are well known in the art and can include administration of "naked DNA" as well as a nucleic acid associated with a carrier such as a cationic or anionic liposome, or polylysine. (See, e.g. *Brigham et al.* Amer. J. Respir. Cell and Mol. Biol. 8:209-213 (1993); *Felgner et al.* Proc. Nat. Acad. Sci. USA 84:7413; and U.S. Patent No. 4,897,355 (*Eppstein et al.*)).

Also provided is a method of producing an antiangiogenic protein comprising administering to a cell a virus comprising a recombinant nucleic acid encoding an antiangiogenic protein inserted within the viral nucleic acid, wherein the recombinant nucleic acid can be packaged in a virus particle, and whereby expression of the recombinant nucleic acid produces the antiangiogenic protein. This method can therefore be used to produce antiangiogenic proteins which are secreted extracelluary in which the antiangiogenic protein can be harvested from the extracellular medium as well as those antiangiogenic proteins which are not secreted from the cell, in which the cell may have to be disrupted, possibly including additional disruption of the cell membrane. In a preferred embodiment, the antiangiogenic proteins are secreted readily from the cells. Protein harvesting methods are standard in the art and exemplified herein. The antiangiogenic protein can be purified to any desired level of purity, as is also standard in the art.

Also provided is a method of screening an antiangiogenic protein for bioactivity, comprising administering to a first cell a virus containing a recombinant nucleic acid encoding the antiangiogenic protein, wherein the first cell expresses the recombinant nucleic acid encoding the antiangiogenic protein and thereby produces the antiangiogenic protein, contacting a second cell with the antiangiogenic protein, and monitoring the second cell for a biological response to the antiangiogenic protein, thereby screening the antiangiogenic protein for bioactivity.

This method can therefore screen particular antiangiogenic proteins for a particular bioactivity as discussed above, such as reduction in tumor mass, reduction of tumor vascularization, an increase in tumor necrosis, and reduction of tumor cellular proliferation.

The screening method may entail harvesting the antiangiogenic protein from the first cell which produces the antiangiogenic protein before the antiangiogenic protein is administered to a second cell which is then monitored for a biological response to the antiangiogenic protein, or the method may entail admixing the first cell which produces the antiangiogenic protein with the second cell which is then monitored for a biological response to the antiangiogenic protein. Similarly, the first cell which produces the antiangiogenic protein may be the same cell type as the second cell or the first and second cells may be different cell types.

The present invention also discloses a method of screening an antiangiogenic protein for bioactivity comprising administering to a cell an adenovirus containing a recombinant nucleic acid encoding the antiangiogenic protein, wherein the cell expresses the recombinant nucleic acid encoding the antiangiogenic protein and thereby produces the antiangiogenic protein, and monitoring the cell for a biological response to the antiangiogenic protein, thereby screening the antiangiogenic protein for bioactivity. The screening method, therefore, does not require a second cell which may not be producing an antiangiogenic protein, but may be employed using only a cell which is itself producing an antiangiogenic protein.

The screening assays of the invention may conveniently employ the antiangiogenic protein directly from the cell in which it is produced. This is achieved most preferably by simply expressing the selected antiangiogenic protein within the cell, typically a eukaryotic cell, followed by preparing a sample of the cell culture medium which includes the antiangiogenic protein. Alternatively, an additional purification step is accomplished on the culture medium containing the antiangiogenic protein in order to recover a purified quantity of antiangiogenic protein molecules. The additional purification steps may include specific binding of the antiangiogenic protein to a antiangiogenic protein-specific ligand or receptor.

By comparing the binding of the selected effector in the presence or absence of the candidate antiangiogenic protein, one can obtain information regarding the binding properties of the antiangiogenic protein. There are believed to be a wide variety of embodiments which can be employed to determine the effect of the antiangiogenic protein on cells, especially epithelial cells, and the invention is not intended to be limited to any one such method. However, it will generally be desirable to employ a system wherein one can measure the ability of the antiangiogenic protein to bind to and or be modified by the effector employed. One method which may be employed may use a labeled antiangiogenic protein, which has been labeled in a manner such that the label is quantitatively retained in the resultant antiangiogenic protein/receptor complex. A convenient approach is the use of a radioactive label, such as $^{1}125$, $^{14}C$ or $^{3}H$, which may be directly quantitated in both the antiangiogenic protein and the resultant complex. In certain assays, the admixture containing the antiangiogenic protein and a receptor is allowed to incubate for a selected amount of time, and the resultant incubated mixture subjected to a separation means in order to separate the unbound antiangiogenic protein remaining in the admixture from any antiangiogenic protein/receptor complex so produced. Then, one simply measures the amount of each, e.g., versus a control to which no candidate antiangiogenic protein has been added. This measurement can be made at various time points where velocity data is desired. From this, one may determine the ability of the antiangiogenic protein to alter or modify the function of the receptor. Numerous techniques are known which could be employed for the separation of the antiangiogenic protein from an antiangiogenic protein/receptor complex, and all such methods are intended to fall within the scope of the invention. Use of thin layer chromatographic methods (TLC), HPLC, spectrophotometric, gas chromatographic/mass spectrophotometric or NMR analyses. Other, more specific methods of purification already noted (affinity binding or immunoprecipitation) may be used, as well. It is contemplated that any such technique may be employed so long as it is capable of differentiating between the antiangiogenic protein and complex, and can be used to determine enzymatic function such as by identifying or quantifying the substrate and product. The antiangiogenic protein/receptor complex itself may also be the subject of techniques such as x-ray crystallography.

The screening methods described herein can also be utilized to screen an antiangiogenic protein for bioactivity, comprising administering to a first cell a viral nucleic acid encoding the antiangiogenic protein, wherein the first cell expresses the viral nucleic acid encoding the antiangiogenic protein and thereby produces the antiangiogenic protein, contacting a second cell with the antiangiogenic protein, and monitoring the second cell for a biological response to the antiangiogenic protein, thereby screening the antiangiogenic protein for bioactivity.

Similarly, the screening methods described herein can be utilized to screen an antiangiogenic protein for bioactivity comprising administering to a cell a viral nucleic acid encoding the antiangiogenic protein, wherein the cell expresses the viral nucleic acid encoding the antiangiogenic protein and thereby produces the antiangiogenic protein, and monitoring the cell for a biological response to the antiangiogenic protein, thereby screening the antiangiogenic protein for bioactivity.

The specification and example are considered as exemplary only, with a true scope and spirit of the invention being indicated by the accompanying claims.

Efforts have been made to ensure accuracy with respect to numbers (e.g., amounts, temperature, etc.) but some errors and deviations should be accounted for. Unless indicated otherwise, parts are parts by weight, temperature is in °C and pressure is at or near atmospheric.

**EXAMPLE I**

Cloning of murine endostatin Murine endostatin was cloned by isolating RNA from the livers of 3 week old mice and converting the RNA to cDNA using a reverse transcriptase reaction. Forward (gatctctagaccaccatgcatactcatcaggactttcag) and reverse (gatcatcgatctatttggagaaagaggtca) primers were used with this cDNA template to clone the murine endostatin cDNA into a sequencing plasmid (pkmendo-2). The PCR was performed using pfu enzyme with glycerol and DMSO as additives.The PCR conditions were: 94° C 45 sec, 50° C 45 sec, 25 cycles 70° C 2 min. The sequence was confirmed using an automated sequencer.

Cloning of human endostatin Human endostatin was cloned by isolating RNA from a human hemangioendothelioma. This RNA was converted to cDNA using a reverse transcriptase reaction and used as a template for PCR with the same conditions described above. The forward primer was gatctctagaccaccatggttgcgctcaacagccccctgt. The reverse primer

17

was gatcatcgatctactacttggaggcagtcatgaagct. The PCR product was cloned into a sequencing plasmid (pkhendo-2) and the sequence was confirmed using an automated sequencer.

Generation of signal sequence endostatin (ss-endo) The E3/19K adenovirus signal sequence was added to the murine and human endostatin contructs by PCR. A unique forward primer containing the signal sequence as well as a sequence homologous to the 5' end of the endostatin cDNA was created.

Murine primer:

gatctctagaccaccatgaggtacatgattttaggcttgctcgcccttgcggcagtctgcagcgcggcccatactcatca ggactttcag

Human Primer:

gatctctagaccaccatgaggtacatgattttaggcttgctcgcccttgcggcagtctccagcgcggccgttgcgctcaa cagccccctg

For the murine construct, pkmendo-2 was used as a template with the same PCR conditions and reverse primer as described above to generate a PCR product carrying the signal sequence 5' to the cDNA for murine endostatin. This was ligated into a sequencing plasmid resulting in pssmendo-6. The sequence was confirmed with an automated sequencer.

For the human construct, pkhendo-2 was used as a template with the same PCR conditions as described. The PCR product was ligated into a sequencing plasmid to generate psshendo.

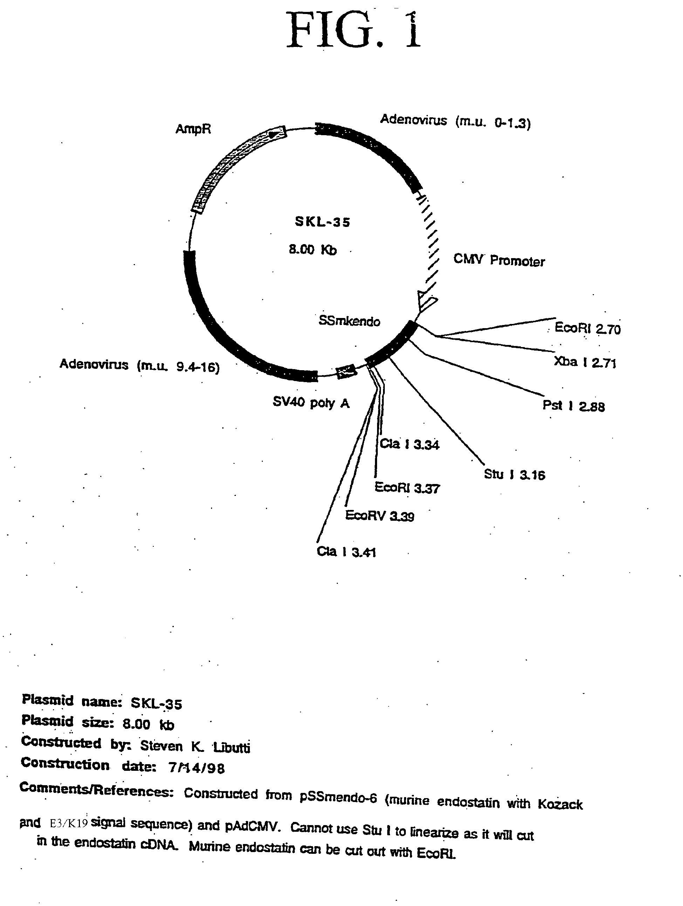

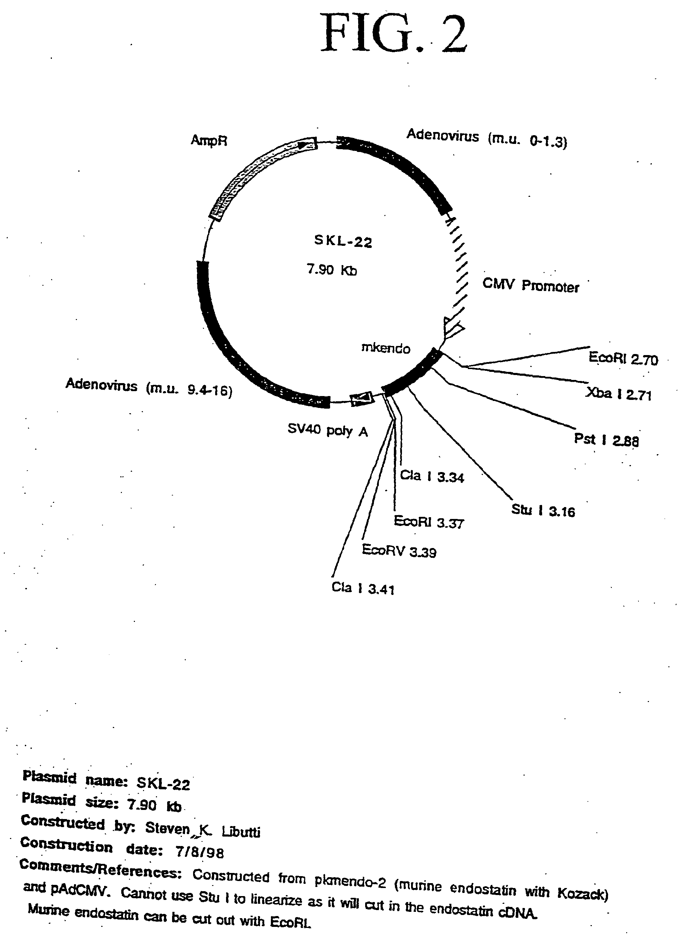

Generation of adenoviral shuttle plasmids Two adenoviral shuttle plasmids were created, one with signal sequence murine edndostatin (pSKL-35) (Fig. 1) and one with murine endostatin without the signal sequence (pSKL-22) (Fig. 2), using the plasmid pAd/CMV.1 as a backbone. The plasmids were created by excising the DNA coding for endostatin (with or without the signal sequence) from the appropriate parent plasmid (pkmendo or pssmendo) using Eco R1. This Eco- R1 fragment was ligated into the shuttle vector and the orientation confirmed by restriction digest. Recombinant adenovirus was then generated using standard techniques.

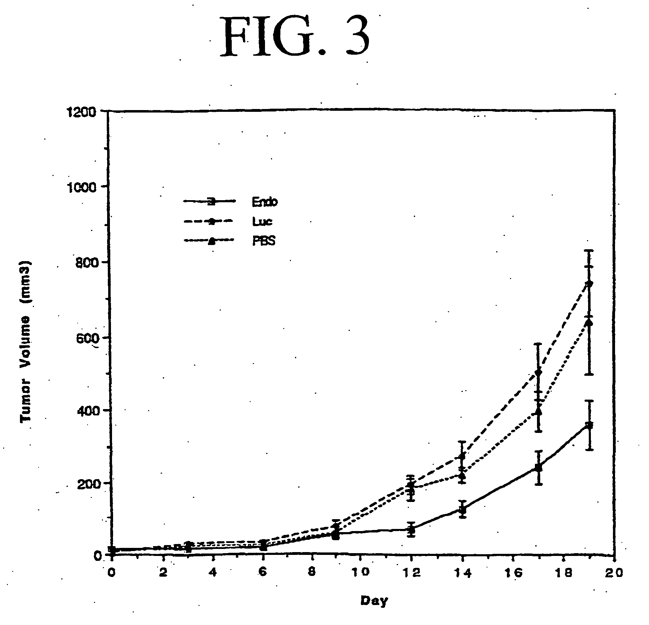

Anti-tumor effect of Ad-ss-mEndo in vivo Six- to 8-week old nude mice were injected subcutaneously with $10^6$ Lewis lung carcinoma cells in 200 ml PBS on day -4. Mice were treated intraperitoneally with 1 X $10^9$ plaque-forming units (pfu) of adenovirus encoding endostatin (Endo, n=8), adenovirus encoding luciferase (Luc, n=6) or no adenovirus (PBS, n=5) in 2 mL PBS on days 0, 7, and 14. Tumors were measured approximately every 3 days by an investigator that did not know the treatment regimes of the individual animals, and tumor volume was calculated using the formula: volume = width$^2$ x length x 0.52. Animals treated with endostatin had significantly smaller tumors than control animals from day 12 until the end of the experiment on day 19 (Fig. 3) (p<0.02, Kruskal-Wallis).

Supernatant endostatin levels after infection with adenovirus

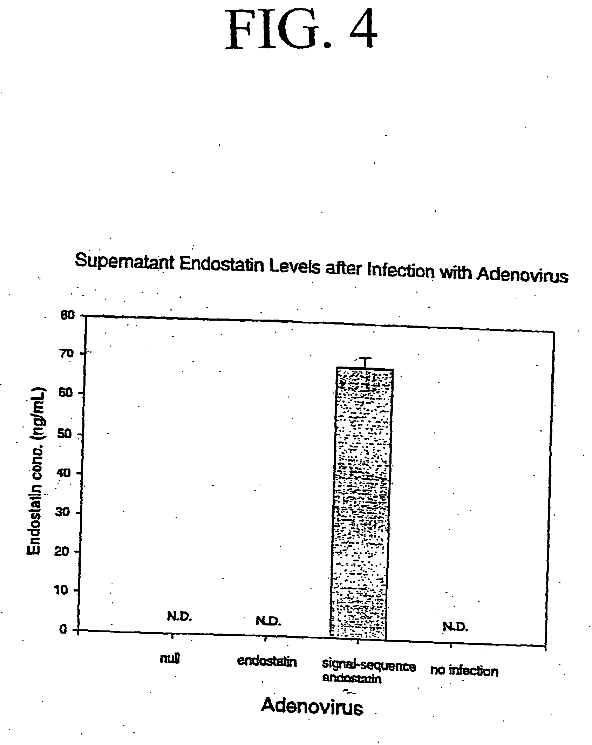

1 x $10^7$293 (E1-transformed human embryonic kidney) cells were plated in 10-cm culture dishes in DMEM containing 10% FCS, glutamine, penicillin, streptomycin, gentamicin, and fungizone and incubated overnight. Cells then were infected with $10^9$ pfu (MOI=100) of wild-type adenovirus (null), adenovirus containing the murine endostatin gene, adenovirus containing the signal-sequence endostatin construct, or no adenovirus in 10 ml of the same medium and incubated for 24 h. Supernatants then were harvested and centrifuged at 5000 rpm for 5 min to remove cell debris. Each infection was performed in triplicate, and supernatant endostatin concentrations were determined for each infection in duplicate, using a competitive immunoassay (Cytimmune, College Park, MD). Endostatin was not detectable (ND) in the supernatants from the null, endostatin, or uninfected plates, but was present at a concentration of 68.5 $\pm$ 2.8 ng/mL (mean $\pm$ S.D.) in the supernatants from signal-sequence endostatin-infected plates (Fig. 4).

Supernatant endostatin levels after infection with adenovirus Experimental design was identical to the previous experiment with the following exceptions: $10^6$ cells/well were plated in 6-well plates and were infected with $10^8$ pfu (MOI=100) of adenovirus in 1.0 ml of medium.

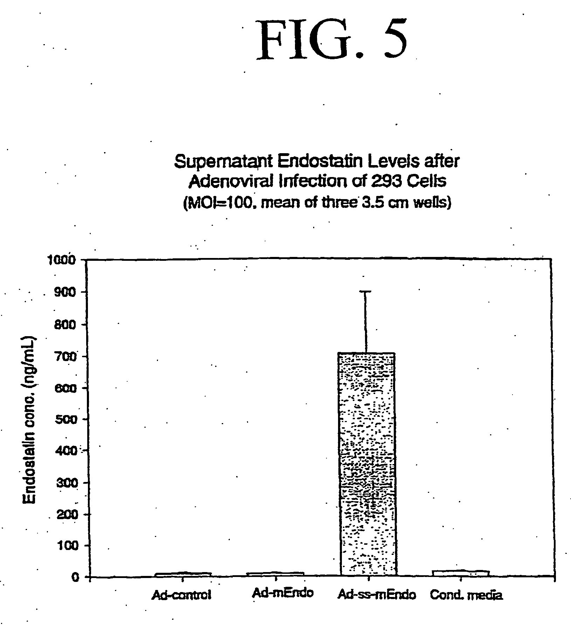

Endostatin concentrations detected in the Ad-control or Ad-mEndo-infected wells were lower than that in conditioned media from uninfected 293 cells (16.3 $\pm$ 1.3 ng/mL). Endostatin concentration was 705.7 $\pm$ 191.4 in the Ad-ss-mEndo-infected wells (Fig. 5) (concentrations expressed as mean $\pm$ S.D.). Immunoreactivity in the control wells was similar to that of 10% FCS alone, suggesting the presence of a protein in bovine serum that cross-reacts with polyclonal anti-murine endostatin antibody.

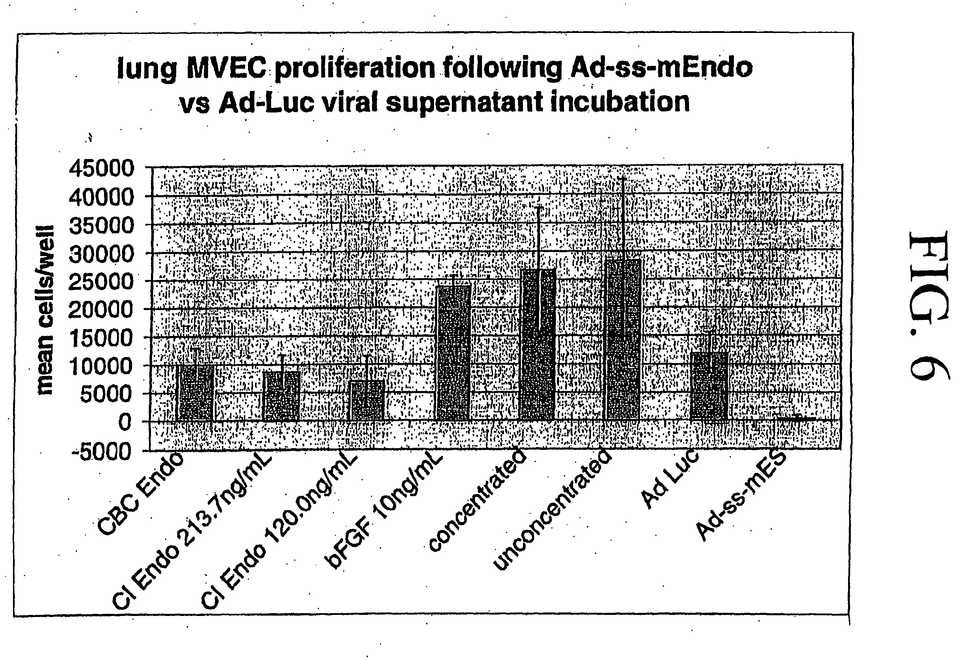

Inhibition of microvascular endothelial cell proliferation using Ad-ss-mEndo To assess the in vitro function of adenovirally produced endostatin, the proliferation of human lung microvascular endothelial cells (MVEC) was examined in the presence of supernatant from cells infected with Ad-ss-mEndo produced as described in the previous experiment. On Day -1, 1 x $10^5$ MVEC were plated in each well of 12-well plates in EGM-2 endothelial cell growth medium. On Day 0, and again on Day 4, cells were treated with supernatants from cells infected with Ad-ss-mEndo, a control adenovirus, or no adenovirus (concentrated or unconcentrated supernatant). In addition recombinant endostatin obtained from Calbiochem (CBC) or Cytimmune Sciences (CI) were tested. All wells contained a total volume of 0.5 ml, and 10 ng/ml basic fibroblast growth factor (bFGF) as a stimulant. In the Ad-ss-mEndo wells, the mean number of cells was 333, and two wells showed a complete absence of MVEC (Fig. 6). Negative control wells, including EGM-2 or concentrated or unconcentrated conditioned media contained mean numbers of cells ranging from 23,000 to 28,000. Wells treated with recombinant endostatin yielded mean numbers of cells ranging from 7,000 to 10,000. Wells treated with control virus yielded a mean cell count of 12,000, suggesting some nonspecific adenoviral effect on MVEC proliferation, but not the near complete effect seen with the endostatin construct.

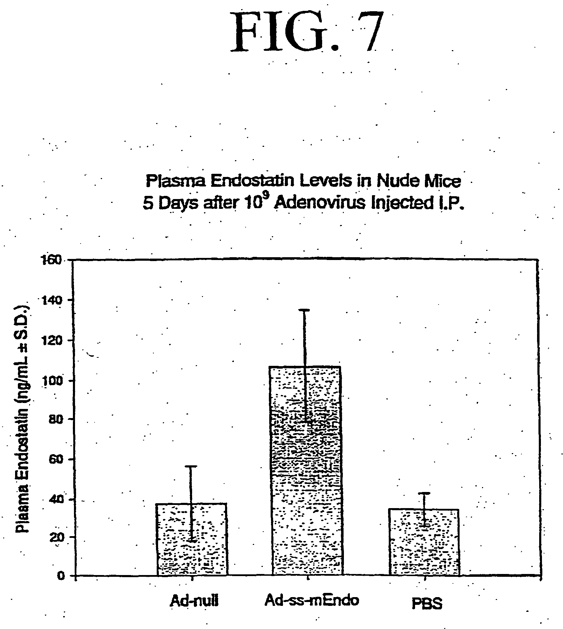

Ad-ssmEndo raises plasma endostatin levels in mice To evaluate the ability of Ad-ss-mEndo to generate endostatin in vivo, nude mice were injected intraperitoneally with $10^9$ pfu of Ad-ss-mEndo, a control adenovirus (Ad-null), or no virus in 2.0 ml saline (PBS). Five days later, plasma samples were analyzed for endostatin concentration by ELISA. Plasma levels in the Ad-ss-mEndo treated animals were 106 $\pm$ 28.0 ng/ml, versus 37 $\pm$ 19.1 ng/ml and 34 $\pm$ 8.5 ng/ml in the Ad-null and PBS groups, respectively (mean $\pm$ S.D.) (Fig. 7).

EXAMPLE II

Cloning of the murine endostatin gene Murine cDNA was obtained by isolating RNA (RNeasy Mini Kit, Qiagen, Valencia, CA) from snap-frozen 2-week-old C57BL/6 mouse (Charles River Laboratories, Wilmington, MA) liver and treating with Moloney murine leukemia virus reverse transcriptase (Life Technologies, Gaithersburg, MD). The murine endostatin gene was cloned into the TA cloning vector (Invitrogen, Carlsbad, CA) by polymerase chain reaction (PCR) using the primers *sense:* GATCTCTAGACCACCATGCATACTCATCAGGACTT and *antisense:* ACTGGAGAAAGAGGTTTATCTAGCTACTAG. The 18-amino acid E3/19K signal sequence (MRYMILGLLALAAVCSAA) was inserted upstream from the endostatin sequence by PCR using the primers *sense:* GATCTCTAGACCACCATGAGGTACATGATTTTAGGCTTGCTCGCCCTTGCGGCAGTCTGCAGCGCGGCCCATAC TCATACTCATCAGGACTTTCAG and *antisense:* as above. Plasmid DNA was amplified in DH5a cells (Life Technologies) and the signal sequence-murine endostatin (ss-mEndo) sequence was confirmed (ABI Prism 3 10 autosequencer. PE Applied Biosystems, Foster City, CA).

Synthesis of adenoviral vectors The ss-mEndo construct was digested with EcoRI and cloned by blunt-end ligation into the multiple cloning site of the adenoviral shuttle plasmid pAd/CMV. 1. The resulting plasmid was recombined with type 5 E I A/B-deleted adenovirus as previously described (6.7) and used to infect 293 cells (American Type Culture Collection. Manassas, VA). Plaque DNA was extracted using proteinase K digestion, phenol extraction, and ethanol precipitation and screened for ss-mEndo by PCR. The resulting virus, Ad-ss-mEndo, was amplified in 293 cells. A similar strategy was used to create control recombinant viruses containing the genes for P-galactosidase (Ad-p-gal) and firefly luciferase (Ad-luc). Viruses were titered using a standard plaque forming assay in 293 cells.

*In vitro* infection with recombinant adenovirus Cells were grown in complete medium consisting of DMEM with 10% fetal calf serum, 100 U/mL penicillin, 100$\mu$g/mL streptomycin, 50 $\mu$g/mL gentamicin, 0.5 ~$\mu$g/mL Fungizone, and 4 mM glutamine (Biofluids, Rockville, MD). Cells were infected at a multiplicities of infection (MOIs) ranging from 0. 1 to 100 (105 to 108 plaque-forming units [pfu] per 106 cells in 1.0 mL complete media) with Ad-ss-mEndo, Ad-luc, or no virus, and incubated at 37 °C for 24 h. Supernatants were centrifuged at 2 x g for 5 min and assayed for endostatin using a competitive enzyme immunoassay (EIA)(Cytimmune Sciences, College Park, MD) according to the manufacturer's instructions. 293 cell supernatants were concentrated ten-fold in cellulose columns (Centricon YM-10, Millipore, Bedford, MA) and analyzed by Western blotting (NuPAGE, Novex, San Diego, CA) using 570 ng/mL rabbit antimurine endostatin polyclonal IgG antibody (gift of Cytimmune Sciences). The EIA murine endostatin standard was used as a positive control. The susceptibility of the murine colon adenocarcinoma cell line MC38 (developed in the Surgery Branch, National Cancer Institute) to adenoviral infection was tested by infecting cells with Ad-βgal as described above and assaying for β-gal 24 h later using a staining kit (BoehringerMannheim, Indianapolis, IN). Susceptibility of the murine hepatocyte line NMuLi (American Type Culture Collection) to Ad-β-gal infection was used as a positive control.

Functional assay of virally generated endostatin The human melanoma cell line 501 Mel (Z.-H. Wang, National Cancer Institute) was infected as above with varying MOIs of Ad-ss-mEndo or Ad-luc. Supernatants were harvested 24 h later and centrifuged as above. Supernatants were analyzed for their ability to inhibit endothelial cell proliferation as previously described (8), with slight modifications. Briefly, 1000 bovine capillary endothelial cells (EJG, American Type Culture Collection) were plated in complete medium in each well of collagen 1-coated 96-well plates (Biocoat, Becton Dickinson, Bedford, MA). After overnight incubation at 37 °C, the medium as aspirated and replaced with 20. ~tL of the supernatant sample to be tested. Eight samples of each supernatant were tested. After 20 min incubation at 37°C, 80 ~lL of modified complete medium containing 5% fetal calf serum and I ng/mL basic fibroblast growth factor (R&D Systems, Minneapolis, MN) was added. After 72 h incubation at 37°C, proliferation was analyzed by WST- I assay (Boehringer Mannheim) according to the manufacturer's instructions. Inhibition of proliferation in each sample was calculated according to the formula:

$$\text{Inhibition (\%)} = (\text{Mean A}_{450(\text{control})} - \text{A}_{450(\text{sample})})/\text{mean A}_{450(\text{control})} \times 100;$$

where $A_{450}$ is the absorbance at 450 nm measured in a Multiskan MCC/340 plate reader (Titertek, Huntsville, AL), and control represents uninfected cell supernatant.

<u>In vivo production of endostatin</u> Animal experiments were conducted according to protocols approved by the National Institutes of Health Animal Care and Use Committee. Eight-week-old female nude mice (Charles River Laboratories) were injected intravenously by lateral tail vein with $10^9$ or $10^{10}$ pfu Ad-ss-mEndo, or no virus, in 100 $\mu$L phosphate buffered saline (PBS)(n=5 animals per group) in order to assess dose response and toxicity. Animal well-being was monitored for 6 days, and surviving mice were euthanized and autopsied. To assess the duration and amount of endostatin expression, mice were injected with $10^9$ pfu of Ad-ss-mEndo or Ad-luc, or no virus, as above on day 0. On days 2, 4, 8, and 13, mice were euthanized with carbon dioxide and blood samples obtained by cardiac puncture (n=6 animals per treatment group per time point). Samples were centrifuged in EDTA-containing tubes (Microtainer, Becton Dickinson, Franklin Lakes, NJ) and plasma endostatin levels were determined by EIA.

<u>In vivo activity of endostatin</u> In a treatment experiment, mice were injected subcutaneously with 106 MC38 cells in 200 ViL PBS. Two days later (treatment day 0), and again I week later (treatment day 7), mice were injected with 109 pfu of Ad-ss-mEndo or Ad-luc, or no virus as described above (n= 10 animals per treatment group). Tumors were measured in two dimensions using calipers on days 0, 2, 5, 7, 9, 11, and 13 by an investigator blinded to the treatment groups. Tumor volume was calculated according to the formula: *volume = width$^2$ x length x 0.52.*

<u>Statistical analysis</u> Data are presented as the mean $\pm$ S.E. Comparisons between groups were performed using the Mann-Whitney U test (Instat 2.01, GraphPad Software), and two-tailed p values less than 0.05 were considered significant.

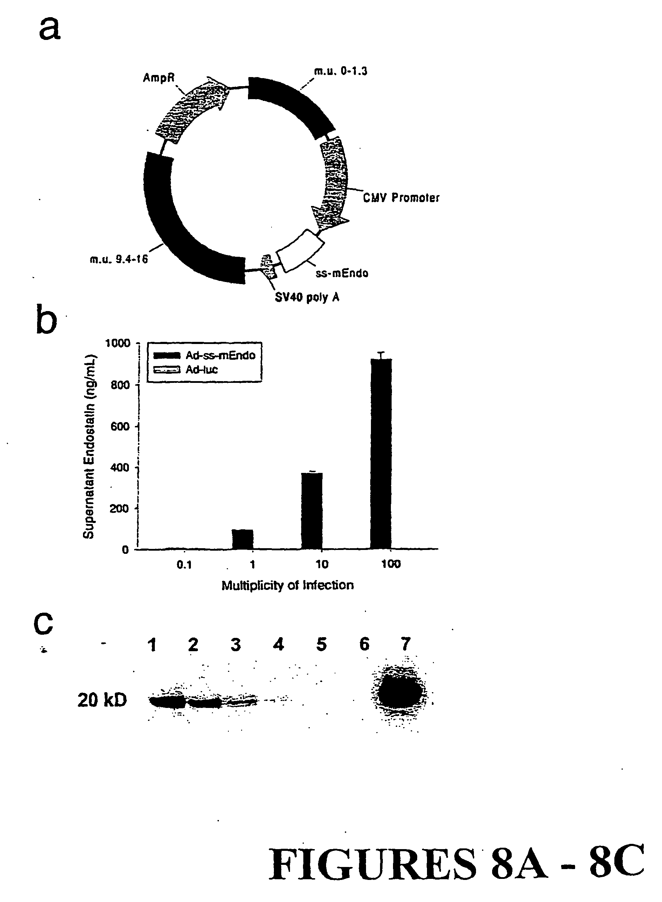

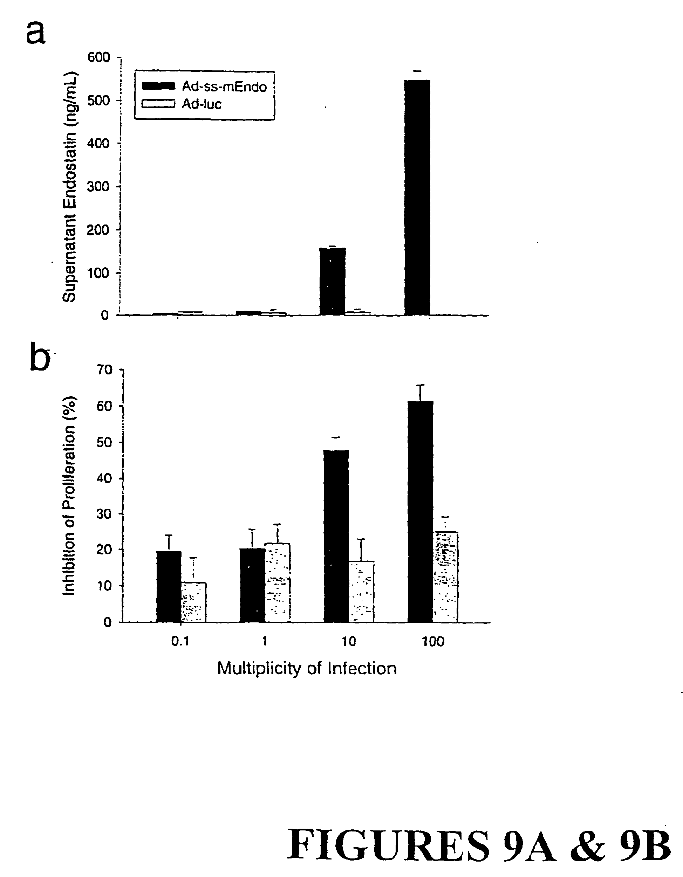

As stated above, the murine endostatin gene preceded by the E3/19K signal sequence (14) was cloned into an adenoviral shuttle plasmid and recombinant Ad-ss-mEndo was generated (Figure 8a). Infection of 293 cells (in which E I -deleted adenovirus can replicate) yielded dose-dependent elevation of supernatant endostatin concentrations, up to 920 $\pm$ 33 ng/mL at an MOI of 100 (Figure 8b). Western blotting of the Ad-ss-mEndo-infected cell supernatant revealed a single band with mobility identical to that of recombinant murine endostatin (Fig. 8c). Human melanoma cells (in which E I -deleted adenovirus cannot replicate) were then infected with Ad-ss-mEndo or Ad-luc. Supernatant endostatin levels (Fig. 9a), and the ability of the supernatant samples to inhibit endothelial cell proliferation (Fig. 9b) were evaluated. A dose dependent increase in inhibition was observed, up to 61 $\pm$ 4% (versus 25 $\pm$ 4% in supernatant from Ad-luc-infected cells, p= 0.0006).

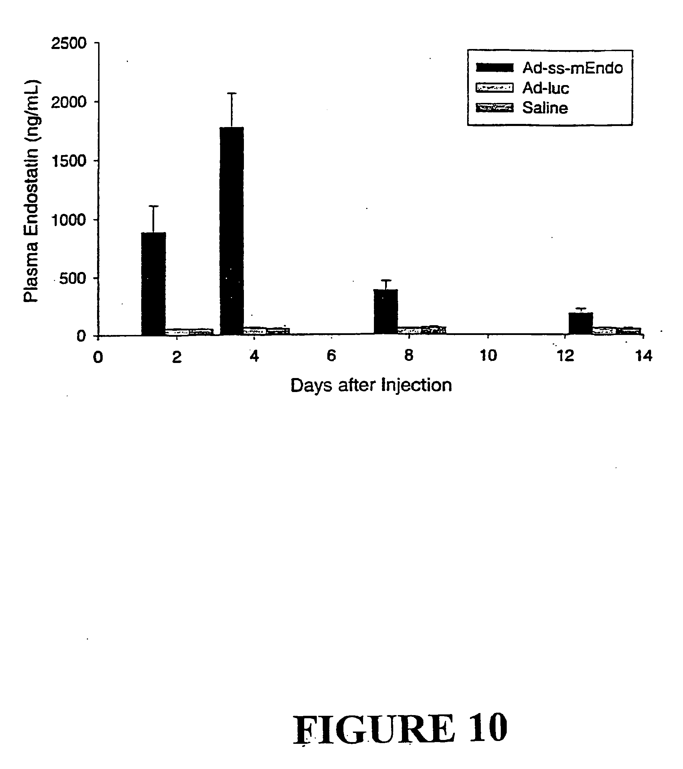

The ability of Ad-ss-mEndo to generate endostatin *in vivo* was then assessed. Mice receiving $10^9$ pfu Ad-ss-mEndo demonstrated 100% survival and appeared healthy. Autopsy revealed only mild hepatomegaly. After receiving $10^{10}$ pfu, 60% of mice died. Surviving mice were markedly lethargic, and at autopsy revealed massive hepatomegaly with extensive macronodular changes and bilious ascites. Other organs revealed no signs of toxicity. These findings are consistent with the known hepatotoxicity of high-dose adenovirus (15). Therefore, circulating endostatin levels were assessed after a dose of $10^9$ pfu (Fig. 10). Baseline plasma endostatin levels were similar between Ad-luc- and saline treated animals at all time points (mean of all time points, 55 ng/mL and 57 ng/mL, respectively). Peak expression after Ad-ss-mEndo infection was observed 4 days after injection, at which time plasma concentrations were 1770 $\pm$ 292 ng/mL (range, 1180 ng/mL to 3137 ng/mL). On day 13, plasma levels remained significantly elevated (187 39 ng/mL versus 56 $\pm$ 12 ng/mL in Ad-luc-treated animals; p=0.041). The peak circulating levels achieved were 50- to 200-fold higher than those previously reported using non-viral vectors.

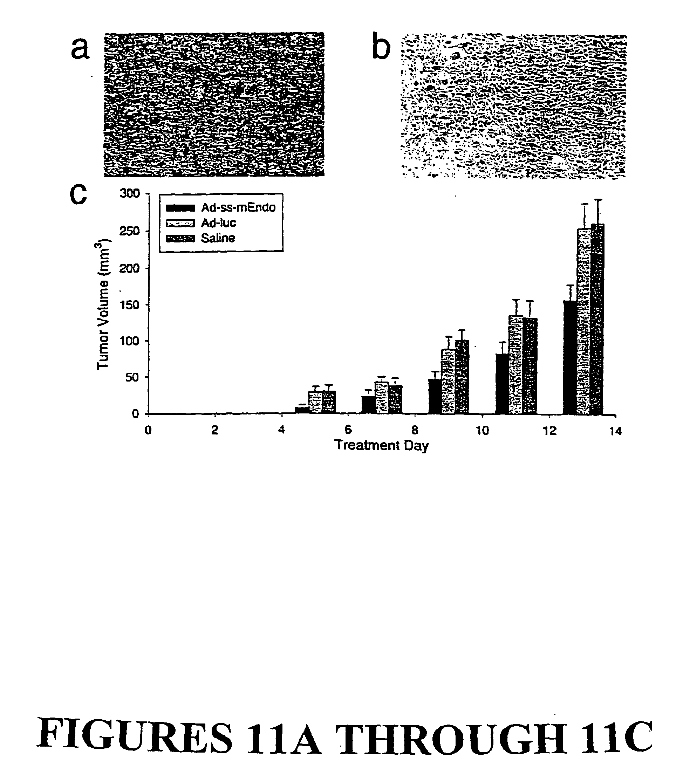

In order to best evaluate the principle of systemic, rather than tumor-directed antiangiogenic gene therapy, a murine tumor cell line that is relatively resistant to adenoviral infection and gene expression was selected. While murine hepatocytes were easily infected with Ad-$\beta$-gal at an MOI of 100 (Fig. 11a), only occasional MC38 adenocarcinoma cells demonstrated blue staining (Fig. 11b). In a two-week *in vivo* treatment model, two weekly treatments were given, based on the time course of endostatin expression presented above. There was no significant difference in tumor size between Ad-luc- and saline-treated animals at any time point (Fig. 11c). Mean tumor size of the Ad-ss-mEndo-treated animals was smaller than that of either control group from day 5 onward. This difference was most marked at the conclusion of the experiment (day 13), at which point 40% tumor growth inhibition compared to saline-treated animals was observed (p=0.012). No toxicity was observed in any of the animals.

This study represents the first *in vivo* report of endostatin gene therapy using an adenoviral vector as inhibition of tumor growth associated with high circulating endostatin levels was demonstrated in a tumor model relatively resistant to tumor-directed gene transfer.

EXAMPLE III

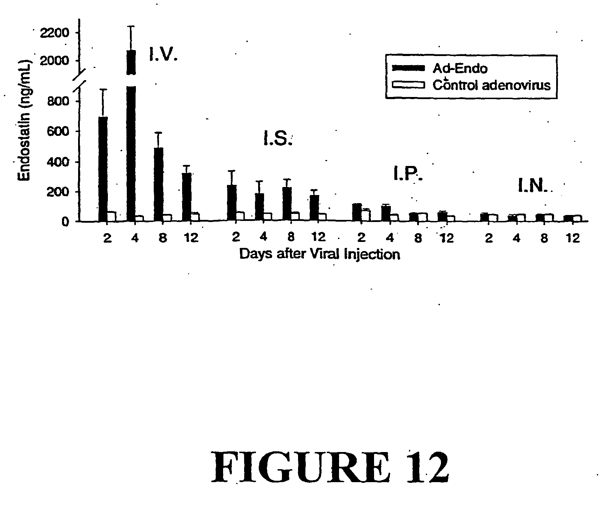

<u>Modes of administration</u> To optimize circulating endostatin levels, four routes of administration of an adenoviral vector carrying the murine endostatin gene (Ad-Endo) were compared. C57BL/6 mice (n=5 to 6 per group for each time point) were treated with $10^9$ plaque-forming units of Ad-Endo or a control adenovirus by intravenous (I.V.), intraperitoneal (I.P.), intrasplenic (I.S. via laparotomy) or intranasal (I.N.) administration. Plasma endostating levels were determined using a competitive immunoassay 2, 4, 8 and 12 days after injection. Baseline plasma levels in control mice were similar for all routes of administration (mean 47 ng/ml). I.V. virus yielded the highest endostatin levels (>2000 ng/ml), which remained significantly elevated through day 12 (321 vs. 48 ng/mL, p=0.002, Mann-Whitney U-test) (Figure 12). I.S. injection yielded endostatin levels higher than control, but lower than those achieved with I.V. injection. I.P. and I.N. injection had minimal or no effect on endostatin levels. Therefore, recombinant adenovirus carrying a murine endostating transgene can lead to sustained, potentially therapeutic circulating levels of endostatin in mice, with the highest levels occurring after I.V. administration.

EXAMPLE IV

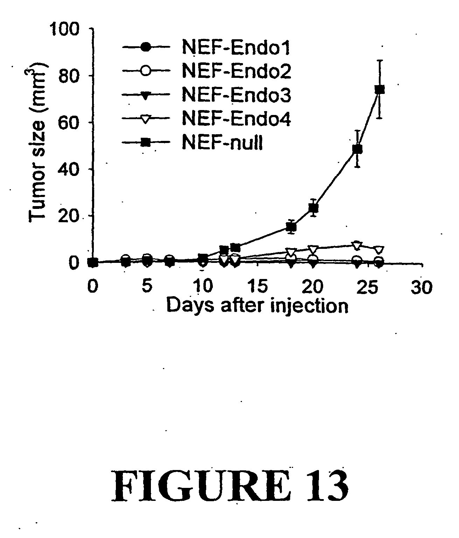

<u>Retroviral transduction of neoplastic murine hepatocytes with endostatin</u> Neoplastic murine hepatocyte cell line, NmuLi was transduced with the murin endostatin gene to test the ability of antiangiogenic gene transfer to inhibit tumor growth. Replication incompetent retroviruses were produced using the pEF-null plasmid. The endostatin gene was cloned from mouse liver and cloned into pEF-null to create pEF-Endo. NmuLi cells were transduced with pEF-null or pEF-Endo-derived retrovirus and selected in G418. Four endostating-transduced clones were amplified and supernatant endostatin assayed by Western blot and competitive enzyme immunoassay. Athymic nude mice (n=8 per group) were injected S.Q. with $5 \times 10^5$ NmuLi cells, pEF-null transduced cells (NEF-null), or clones expressing variable amounts of endostatin (NEF-Endo 1 to 4). Tumor volumes were calculated by the formula $v = w^2 \times 1 \times \pi/6$. NEF-Endo clones yielded supernatant endostatin levels up to 223 ng/mL (versus 20 ng/ml in NEF-null supernatant)(Figure 13). Twenty-six days after injection, NEF-null tumors were significantly larger than tumors derived from NEF-Endo clones (p<0/0001, Kruskal-Wallis test). Retroviral transduction of neoplastic murine hepatocytes with the endostatin gene leads to secretion of functional endostatin sufficiently active to inhibit tumor growth.

**References**

1. Folkman, J. Angiogenesis in cancer, vascular, rheumatoid and other disease. Nature Med., 1: 27-311995.

2. Crystal, R.G. The body as a manufacturer of endostatin. Nature Biotechnol., 17: 336-337, 1999

3. Folkman, J. Antiangiogenic gene therapy. Proc. Natl. Acad. Sci. USA, 95: 9064-9066,1998.

4. Kong, H.-L., and Crystal, R.G. Gene therapy strategies for tumor antiangiogenesis. J. Natl. Cancer Inst., 90: 273-286, 1998.

5. Lin, P., Buxton, J.A., Acheson, A., Radziejewski, C., Maisonpierre, P.C., Yancopoulos, G.D., Channon, K.M., Hale, L.P., Dewhirst, M.W., George, S.E., and Peters, K.G. Antiangiogenic gene therapy targeting the endothelium-specific receptor tyrosine kinase Tie2. Proc. Natl. Acad. Sci. USA, 95: 8829-8834, 1998.

6. Katayose, D., Gudas, J., Nygeun, H., Cowan, K., and Seth, P. Cytotoxic effects of adenovirus-mediated wild type p53 protein expression in normal and tumor mammary epithelial cells. Clin. Cancer Res. 1: 889-898, 1995.

7. Katayose, D., Wersto, R., Cowan, K., and Seth, P. Effects of a recombinant adenovirus expressing WAF/Cip I on cell growth, cell cycle, and apoptosis. Cell Growth Differ., 6: 1207-1212, 1995.

8. Blezinger, P., Wang, J., Gondo, M., Quezada, A., Mehrens, D., French, M., Singhal, A., Sullivan, S. Rolland, A., Ralston, R., and Min, W. Systemic inhibition of tumor growth and tumor metastases by intramuscular administration of the endostatin gene. Nature Biotech., 17: 343-348, 1999.

9. Cao, Y. Endogenous angiogenesis inhibitors: angiostatin, endostatin, and other proteolytic fragments. Prog. Mol. Subcell. Biol., 20: 161-176, 1998.

10. O'Reilly, M.S., Boehm, T., Shing, Y., Fukai, N., Vasios, G., Lane, W.S., Flynn, E., Birkhead, J.R., Olsen, B.R., and Folkinan, J. Endostatin: an endogenous inhibitor of angiogenesis and tumor growth. Cell, 88: 277-285, 1997.

11. Chen, Q.-R., Kumar, D., Stass, S.A., and Mixson A.J. Liposomes complexed to plasmids encoding angiostatin and endostatin inhibit breast cancer in nude mice. Cancer Res., 59.- 3308-3312, 1999.

12. Ferry, N. and Heard, J. Liver-directed gene transfer vectors. Hum. Gene. Ther., 9: 1975-1981, 1998.

13. Tomko, R., Xu, R., and Philipson, L. HCAR and MCAR: the human and mouse cellular receptors for subgroup C adenoviruses and group B Coxsackieviruses. Proc. Natl. Acad. Sci. USA, 94: 3352-3354, 1997.

14. Persson, H., Jornvall, H., and Zabielski, J. Multiple mRNA species for the precursor to an adenovirus-encoded glycoprotein: identification and structure of the signal sequence. Proc. Natl. Acad. Sci. USA. 77: 6349-6353, 1980.

15. Hogaboam, C.M.. Simpson, K.J.. Chensue, S.W., Steinhauser, M.L., Lukacs, N.W., Gauldie, J., Stricter. R.M., and Kunkel, S.L. Macrophage inflammatory protein-2 gene therapy attenuates adenovirus and acetaminophen-mediated hepatic injury. Gene Ther., 6: 573-584, 1999

**Claims**

1. A compound comprising a recombinant nucleic acid encoding an antiangiogenic protein operatively linked to an adenovirus E3/19K signal sequence inserted within a viral nucleic acid, wherein the recombinant nucleic acid can be packaged in a virus particle and wherein expression of the recombinant nucleic acid encoding the antiangiogenic protein results in production of the antiangiogenic protein.

2. The compound of claim 1, wherein the viral nucleic acid comprises an adenovirus nucleic acid.

3. The compound of claim 1, wherein the viral nucleic acid comprises a retroviral nucleic acid.

4. The compound of claim 1, wherein the antiangiogenic protein comprises endostatin.

5. The compound of claim 1, wherein the antiangiogenic protein comprises thrombospondin.

6. The compound of claim 1, wherein the antiangiogenic protein comprises EMAP-II

7. The compound of claim 1, wherein the antiangiogenic protein comprises IP-10.

8. The compound of claim 1, wherein the antiangiogenic protein comprises angiostatin.

9. The compound of claim 1, wherein the antiangiogenic protein comprises vasostatin.

10. The compound of claim 1, wherein the antiangiogenic protein comprises vasculostatin.

11. The compound of claim 1, wherein the antiangiogenic protein comprises IL-12.

12. The compound of claim 1, wherein the antiangiogenic protein comprises platelet factor 4.

13. The compound of claim 1, wherein the antiangiogenic protein comprises cleavage products of collagen VIII.

14. The compound of claim 1, wherein the antiangiogenic protein comprises cleavage products of collagen XV.

15. The compound of claim 1, wherein the antiangiogenic protein comprises restatin.

16. The compound of claim 2, wherein the recombinant nucleic acid is replication deficient.

17. The compound of claim 3, wherein the recombinant nucleic acid is replication deficient.

18. An adenovirus comprising the compound of claim 2.

19. A retrovirus comprising the compound of claim 3.

20. The compound of claim 1, wherein the signal sequence encodes an amino acid sequence comprising the amino acid sequence MRYMILGLLALAAVCSAA.

21. Use of a viral vector comprising a recombinant nucleic acid encoding an antiangiogenic protein operatively linked to an adenoviral E3/19K signal sequence to prepare a formulation for systemic administration of an antiangiogenic protein to a subject.

22. The use of claim 21, wherein the viral vector is an adenoviral vector.

**23.** The use of claim 21, wherein the viral vector is a retroviral vector.

**24.** The use of claim 21, wherein the antiangiogenic protein comprises endostatin.

**25.** The use of claim 21, wherein the signal sequence encodes an amino acid sequence comprising the amino acid sequence MRYMILGLLALAAVCSAA.

**26.** The use of claim 21, wherein the formulation is for intravenous administration.

**27.** A use of a viral vector comprising a recombinant nucleic acid encoding an antiangiogenic protein operatively linked to an adenovirus E3/19K signal sequence for preparing a formulation for systemic administration for treating a tumor in a subject.

**28.** The use of claim 27, wherein the viral vector is an adenoviral vector.

**29.** The use of claim 27, wherein the viral vector is a retroviral vector.

**30.** The use of claim 27, wherein the antiangiogenic protein comprises endostatin.

**31.** The use of claim 27, wherein the sequence encodes an amino acid sequence comprising the amino acid sequence MRYMILGLLALAAVCSAA.

**32.** The use of claim 29, wherein the formulation is for intravenous delivery.

**Patentansprüche**

**1.** Verbindung enthaltend eine rekombinante Nukleinsäure, die ein antiangiogenes Protein kodiert, das operativ mit einer Adenovirus-E3/19K-Signalsequenz verbunden ist, die in eine virale Nukleinsäure insertiert ist, wobei die rekombinante Nukleinsäure in ein Viruspartikel verpackt sein kann und wobei die Expression der rekombinanten Nukleinsäure, die das antiangiogene Protein kodiert, zur Produktion des antiangiogenen Proteins führt.

**2.** Verbindung nach Anspruch 1, wobei die virale Nukleinsäure eine Adenovirus-Nukleinsäure enthält.

**3.** Verbindung nach Anspruch 1, wobei die virale Nukleinsäure eine retrovirale Nukleinsäure enthält.

**4.** Verbindung nach Anspruch 1, wobei das antiangiogene Protein Endostatin enthält.

**5.** Verbindung nach Anspruch 1, wobei das antiangiogene Protein Thrombospondin enthält.

**6.** Verbindung nach Anspruch 1, wobei das antiangiogene Protein EMAP-II enthält.

**7.** Verbindung nach Anspruch 1, wobei das antiangiogene Protein IP-10 enthält.

**8.** Verbindung nach Anspruch 1, wobei das antiangiogene Protein Angiostatin enthält.

**9.** Verbindung nach Anspruch 1, wobei das antiangiogene Protein Vasostatin enthält.

**10.** Verbindung nach Anspruch 1, wobei das antiangiogene Protein Vaskulostatin enthält.

**11.** Verbindung nach Anspruch 1, wobei das antiangiogene Protein IL-12 enthält.

**12.** Verbindung nach Anspruch 1, wobei das antiangiogene Protein Thrombozytenfaktor 4 enthält.

**13.** Verbindung nach Anspruch 1, wobei das antiangiogene Protein Spaltprodukte von Kollagen VIII enthält.

**14.** Verbindung nach Anspruch 1, wobei das antiangiogene Protein Spaltprodukte von Kollagen XV enthält.

**15.** Verbindung nach Anspruch 1, wobei das antangiogene Protein Restatin enthält.

**16.** Verbindung nach Anspruch 2, wobei die rekombinante Nukleinsäure replikationsdefizient ist.

**17.** Verbindung nach Anspruch 3, wobei die rekombinante Nukleinsäure replikationsdefizient ist.

**18.** Adenovirus enthaltend die Verbindung von Anspruch 2.

**19.** Retrovirus enthaltend die Verbindung von Anspruch 3.

**20.** Verbindung nach Anspruch 1, wobei die Signalsequenz eine Aminosäuresequenz kodiert, die die Aminosäuresequenz MRYMILGLLALAAVCSAA enthält.

**21.** Verwendung eines viralen Vektors, der eine rekombinante Nukleinsäure enthält, die ein antiangiogenes Protein kodiert, das operativ mit einer adenoviralen E3/19K-Signalsequenz verbunden ist, um eine Formulierung für die systemische Verabreichung eines antiangiogenen Proteins an ein Individuum herzustellen.

**22.** Verwendung nach Anspruch 21, wobei der virale Vektor ein adenoviraler Vektor ist.

**23.** Verwendung nach Anspruch 21, wobei der virale Vektor ein retroviraler Vektor ist.

**24.** Verwendung nach Anspruch 21, wobei das antiangiogene Protein Endostatin enthält.

**25.** Verwendung nach Anspruch 21, wobei die Signalsequenz eine Aminosäuresequenz kodiert, die die Aminosäuresequenz MRYMILGLLALAAVCSAA enthält.

**26.** Verwendung nach Anspruch 21, wobei die Formulierung für die intravenöse Verabreichung vorgesehen ist.

**27.** Verwendung eines viralen Vektors, der eine rekombinante Nukleinsäure enthält, die ein antiangiogenes Protein kodiert, das operativ mit einer Adenovirus-E3/19K-Signalsequenz verbunden ist, zur Herstellung einer Formulierung zur systemischen Verabreichung, um bei einem Individuum einen Tumor zu behandeln.

**28.** Verwendung nach Anspruch 27, wobei der virale Vektor ein adenoviraler Vektor ist.

**29.** Verwendung nach Anspruch 27, wobei der virale Vektor ein retrovirale Vektor ist.

**30.** Verwendung nach Anspruch 27, wobei das antiangiogene Protein Endostatin enthält.

**31.** Verwendung nach Anspruch 27, wobei die Sequenz eine Aminosäuresequenz kodiert, die die Aminosäuresequnez MRYMILGLLALAAVCSAA enthält.

**32.** Verwendung nach Anspruch 29, wobei die Formulierung zur intravenösen Abgabe vorgesehen ist.

**Revendications**

**1.** Composé comprenant un acide nucléique recombinant encodant une protéine antiangiogénique fonctionnellement liée à une séquence signal $E_3$/19K adénovirale insérée dans un acide nucléique viral, dans lequel l'acide nucléique recombinant peut être encapsidé dans une particule virale et dans lequel l'expression de l'acide nucléique recombinant encodant la protéine antiangiogénique aboutit à la production de la protéine antiangiogénique.

**2.** Composé selon la revendication 1, dans lequel l'acide nucléique viral comprend un acide nucléique adénoviral.

**3.** Composé selon la revendication 1, dans lequel l'acide nucléique viral comprend un acide nucléique rétroviral.

**4.** Composé selon la revendication 1, dans lequel la protéine antiangiogénique comprend l'endostatine.

**5.** Composé selon la revendication 1, dans lequel la protéine antiangiogénique comprend la thrombospondine.

6. Composé selon la revendication 1, dans lequel la protéine antiangiogénique comprend l'EMAP-II.

7. Composé selon la revendication 1, dans lequel la protéine antiangiogénique comprend l'IP-10.

8. Composé selon la revendication 1, dans lequel la protéine antiangiogénique comprend l'angiostatine.

9. Composé selon la revendication 1, dans lequel la protéine antiangiogénique comprend la vasostatine.

10. Composé selon la revendication 1, dans lequel la protéine antiangiogénique comprend la vasculostatine.

11. Composé selon la revendication 1, dans lequel la protéine antiangiogénique comprend l'IL-12.

12. Composé selon la revendication 1, dans lequel la protéine antiangiogénique comprend le facteur plaquettaire 4.

13. Composé selon la revendication 1, dans lequel la protéine antiangiogénique comprend des produits de clivage du collagène de type VIII.

14. Composé selon la revendication 1, dans lequel la protéine antiangiogénique comprend des produits de clivage du collagène de type XV.

15. Composé selon la revendication 1, dans lequel la protéine antiangiogénique comprend la restatine.

16. Composé selon la revendication 2, dans lequel l'acide nucléique recombinant est déficient en matière de réplication.

17. Composé selon la revendication 3, dans lequel l'acide nucléique recombinant est déficient en matière de réplication.

18. Adénovirus comprenant le composé selon la revendication 2.

19. Rétrovirus comprenant le composé selon la revendication 3.

20. Composé selon la revendication 1, dans lequel la séquence signal encode une séquence d'acides aminés comprenant la séquence d'acides aminés MRYMILGLLALAAVCSAA.

21. Utilisation d'un vecteur viral comprenant un acide nucléique recombinant encodant une protéine antiangiogénique fonctionnellement liée à une séquence signal $E_3$/19K adénovirale en vue de la préparation d'une composition destinée à l'administration systémique d'une protéine antiangiogénique à un sujet.

22. utilisation selon la revendication 21, le vecteur viral étant un vecteur adénoviral.

23. Utilisation selon la revendication 21, le vecteur viral étant un vecteur rétroviral.

24. Utilisation selon la revendication 21, la protéine antiangiogénique comprenant l'endostatine.

25. Utilisation selon la revendication 21, la séquence signal encodant une séquence d'acides aminés comprenant la séquence d'acides aminés MRYMILGLLALAAVCSAA.

26. Utilisation selon la revendication 21, la composition étant destinée à l'administration par voie intraveineuse,

27. Utilisation d'un vecteur viral comprenant un acide nucléique recombinant encodant une protéine antiangiogénique fonctionnellement liée à une séquence signal $E_3$/19K adénovirale en vue de la préparation d'une composition destinée à l'administration par voie systémique à un sujet pour traiter une tumeur chez celui-ci.

28. Utilisation selon la revendication 27, le vecteur viral étant un vecteur adénoviral.

29. Utilisation selon la revendication 27, le vecteur viral étant un vecteur rétroviral.

30. Utilisation selon la revendication 27, la protéine antiangiogénique comprenant l'endostatine.

**31.** Utilisation selon la revendication 27, la séquence encodant une séquence d'acides aminés comprenant la séquence d'acides aminés MRYMILGLLALAAVCSAA.

**32.** Utilisation selon la revendication 29, la composition étant destinée à une administration par voie intraveineuse.

# FIG. 1

Plasmid name: SKL-35

Plasmid size: 8.00 kb

Constructed by: Steven K. Libutti

Construction date: 7/14/98

Comments/References: Constructed from pSSmendo-6 (murine endostatin with Kozack and E3/K19 signal sequence) and pAdCMV. Cannot use Stu I to linearize as it will cut in the endostatin cDNA. Murine endostatin can be cut out with EcoRI.

# FIG. 2

Plasmid name: SKL-22

Plasmid size: 7.90 kb

Constructed by: Steven K. Libutti

Construction date: 7/8/98

Comments/References: Constructed from pkmendo-2 (murine endostatin with Kozack) and pAdCMV. Cannot use Stu I to linearize as it will cut in the endostatin cDNA. Murine endostatin can be cut out with EcoRI.

# FIG. 3

# FIG. 4

Supernatant Endostatin Levels after Infection with Adenovirus

Endostatin conc. (ng/mL)

80
70
60
50
40
30
20
10
0

N.D.          N.D.                              N.D.

null        endostatin    signal-sequence   no infection
                          endostatin

Adenovirus

# FIG. 5

Supernatant Endostatin Levels after
Adenoviral Infection of 293 Cells
(MOI=100, mean of three 3.5 cm wells)

lung MVEC proliferation following Ad-ss-mEndo vs Ad-Luc viral supernatant incubation

FIG. 6

# FIG. 7

Plasma Endostatin Levels in Nude Mice
5 Days after 10⁹ Adenovirus Injected I.P.

a

b

c

# FIGURES 8A - 8C

FIGURES 9A & 9B

**FIGURE 10**

FIGURES 11A THROUGH 11C

# FIGURE 12

**FIGURE 13**